(19) **Europäisches Patentamt / European Patent Office / Office européen des brevets**

(11) **EP 1 916 306 B1**

(12) # EUROPEAN PATENT SPECIFICATION

(45) Date of publication and mention
of the grant of the patent:
**29.02.2012 Bulletin 2012/09**

(21) Application number: **06778475.1**

(22) Date of filing: **28.07.2006**

(51) Int Cl.:
*C12N 15/863* (2006.01)          *A61K 39/008* (2006.01)
*A61P 33/02* (2006.01)

(86) International application number:
**PCT/ES2006/070122**

(87) International publication number:
**WO 2007/014977 (08.02.2007 Gazette 2007/06)**

(54) **RECOMBINANT VECTORS BASED ON THE MODIFIED VACCINIA ANKARA (MVA) VIRUS AS VACCINES AGAINST LEISHMANIASIS**

REKOMBINANTE VEKTOREN AUF DER GRUNDLAGE DES MODIFIZIERTEN VACCINIAVIRUS ANKARA (MVA) ALS IMPFSTOFFE GEGEN LEISHMANIASE

VECTEURS RECOMBINES BASES SUR LE VIRUS ANKARA MODIFIE (MVA) UTILISES EN TANT QUE VACCIN CONTRE LA LEISHMANIASIS

(84) Designated Contracting States:
**AT BE BG CH CY CZ DE DK EE ES FI FR GB GR HU IE IS IT LI LT LU LV MC NL PL PT RO SE SI SK TR**

(30) Priority: **30.07.2005 ES 200501886**

(43) Date of publication of application:
**30.04.2008 Bulletin 2008/18**

(73) Proprietor: **CONSEJO SUPERIOR DE INVESTIGACIONES CIENTIFICAS**
**28006 Madrid (ES)**

(72) Inventors:
• **PÉREZ JIMÉNEZ, Eva**
**46021 Valencia (ES)**
• **LARRAGA RODRÍGUEZ DE VERA, Vicente Emilio**
**28040 Madrid (ES)**
• **ESTEBAN RODRÍGUEZ, Mariano,**
**Ctro Nacional de Biotecnología**
**2804 Madrid (ES)**

(74) Representative: **Elzaburu Marquez, Alberto**
**Elzaburu S.L.P.**
**Miguel Angel 21,**
**28010 Madrid (ES)**

(56) References cited:
WO-A1-00/34494          WO-A1-88/06182
WO-A1-92/22327          WO-A1-2004/005523
WO-A2-02/059291          WO-A2-03/047617
ES-A1- 2 171 360          ES-A1- 2 172 482

US-A- 5 212 057

• VAZQUEZ M I ET AL: "Identification of functional domains of the 14-kilodalton envelope protein (A27L) of vaccinia virus" JOURNAL OF VIROLOGY, THE AMERICAN SOCIETY FOR MICROBIOLOGY, US, vol. 73, no. 11, 1 November 1999 (1999-11-01), pages 9098-9109, XP002176445 ISSN: 0022-538X

• GURUNATHAN S ET AL: "Vaccination with DNA encoding the immunodominant LACK parasite antigen confers protective immunity to mice infected with leishmania major" JOURNAL OF EXPERIMENTAL MEDICINE, ROCKEFELLER UNIVERSITY PRESS, JP, vol. 186, no. 7, 6 October 1997 (1997-10-06), pages 1137-1147, XP002969729 ISSN: 0022-1007

• DREXLER I ET AL: "Modified vaccinia virus Ankara as antigen delivery system: how can we best use its potential?" CURRENT OPINION IN BIOTECHNOLOGY, LONDON, GB, vol. 15, no. 6, 1 December 2004 (2004-12-01), pages 506-512, XP004653482 ISSN: 0958-1669

• SUTTER G ET AL: "VACCINIA VECTORS AS CANDIDATE VACCINES: THE DEVELOPMENT OF MODIFIED VACCINIA VIRUS ANKARA FOR ANTIGEN DELIVERY" CURRENT DRUG TARGETS. INFECTIOUS DISORDERS, BENTHAM SCIENCE PUBLISHERS, HILVERSUM, NL, vol. 3, no. 3, 1 September 2003 (2003-09-01), pages 263-271, XP009068362 ISSN: 1568-0053

EP 1 916 306 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- PEREZ-JIMENEZ ET AL: "MVA-LACK as a safe and efficient vector for vaccination against leishmaniasis" MICROBES AND INFECTION, ELSEVIER, PARIS, FR, vol. 8, no. 3, 1 March 2006 (2006-03-01), pages 810-822, XP005398500 ISSN: 1286-4579

- VAZQUEZ ET AL.: 'Identification of functional domains in the 14-kilodalton envelope protein (A27L) of Vaccinia Virus' JOURNAL OF VIROLOGY vol. 73, no. 11, 1999, pages 9098 - 9109, XP002176445

**Description**

**FIELD OF THE INVENTION**

The present invention refers to recombinant viruses based on the modified vaccinia Ankara (MVA) virus. More specifically, the invention refers to recombinant viruses derived from MVA which act as systems for the expression of the LACK protein or immunogenic fragments of the same and their use in the vaccination against leishmaniasis both in human beings as well as in other affected mammals, as dogs.

**STATE OF THE TECHNIQUE**

Leishmaniasis is an anthropozoonosis which includes a complex group of clinical pictures produced by protozoa of the *Leishmania* genus, which act as parasites on cells of the monocyte-macrophage system. *Leishmania* is a flagellated protozoon which belongs to the *Kinetoplastida* order and the *Trypanosomatidae* family. The classification of the *Leishmania* genus into its different species is complex and currently it is carried out by using analysis of the restriction fragments of the DNA of Kinetoplast. It is as follows:

**ORDER: *Kinetoplastida***
**FAMILY: *Trypanosomatidae***
**GENUS: *Leishmania***

**SUB-GENUS: *Leishmania***
**Complex: *L. donovani***

***L. donovani; L. infantum; L. chagasi: L. archibaldi***

**Species outside the *L. donovani* complex**

***L. tropica; L. aethiopica; L. major; L. gerbilli***

**Complex: *L. mexicana***

***L. mexicana; L. amazonensis; L. venezuelensis; L. aristidesi***

**SUB-GENUS: *Viannia***
**Complex: *L. braziliensis***

*L. braziliensis; L. panamensis; L. guyanensis; L. peruviana*

The life cycle is developed in two hosts, one vertebrate (mammal) and an invertebrate vector (female mosquito of the *Phlebotomidae* family, a diptera of the Phlebotomus genus in the Old World and Lutzomyia in the New World, also known as sand fly). In the vertebrate host, *Leishmania* is an obligatory intracellular parasite which is found in macrophages in amastigote form. The amastigotes are round in shape and measure 3-5 $\mu$m x 2-3 $\mu$m, with rudimentary flagella which do not protrude from the soma and are reproduced by binary fission. The amastigotes are passed on by insects, with the bite while sucking blood from the parasite stricken mammal. In the peritrophic sac of the middle intestine of the insect, the amastigotes are transformed into promastygotes, a mobile and elongated form with a single flagellum on its front pole, which are actively multiplied in the middle intestine of the mosquito. 15-20 days after their ingestion they start to detach from the intestinal cuticles and invade the lower pharynx. By biting a new host, the insect inoculates the promastygotes, called metacyclics, which once inside the vertebrate host will be subjected to phagocytosis by the cells of the monocyte-macrophage system, where they will be transformed and multiplied as amastigotes.
The clinical signs of the disease vary depending on the immune response of the host, the strain, and the virulence of the parasite, with cutaneous lesions which cure spontaneously, to the visceral form of the disease, which can lead to death if treatment is not received, being observed.
Cutaneous leishmaniasis is typically caused by *Leishmania tropica, Leishmania major* and *Leishmania aethiopica* (species from the "Old World") and *Leishmania mexicana, Leishmania amazonensis, Leishmania braziliensis, Leishmania panamensis, Leishmania guyanensis, Leishmania peruviana, Leishmania chagasi* and other species in the New World. It is often present in the form of superfcial ulcers with elevated edges, which usually arise in localised or exposed areas of the face and limbs and which can be accompanied by cutaneous lesions and regional adenopathy.

The clinical signs of cutaneous leishmaniasis are similar in the Old and New World. It takes years for the spontaneous resolution of the lesions and it usually leaves an atrophic flat scar.

Visceral leishmaniasis, also known as Kala-azar or Dumdum fever, is caused by the *L. donovani, L. chagasi* and the *L. infantum* species. Its characteristic symptoms in humans consist of, headaches, intermittent fever, asthenia, diarrhoea, abdominal pains, colic, adenopathies, hepatomegaly, splenomegaly, anaemia, leucopoenia, thrombocytopenia, ocular lesions, excessive growth of the nails and eyelashes as well as the appearance of opportunist infections. In Spain and the south-west of Europe, leishmaniasis is a zoonosis, dogs being its main reservoir. Epidemiological studies show that up to 80% of dogs in endemic areas are infected (3, 31), of which 50% develop the visceral form of the disease (3, 17).

The species traditionally known as the cause of visceral leishmaniasis in the Mediterranean area is *Leishmania infantum.* However, its distribution extends to Eastern Europe and countries in Asia. It is currently considered as a synonym of *Leishmania chagasi* (22), as its distribution could extend to Latin America and possibly to the area south of the United States (10).

The World Health Organisation (WHO) has estimated that the world prevalence of the disease in humans is around 12 million people, with an annual incidence of 1.5 to 2 million new cases of cutaneous leishmaniasis and 500,000 new cases of visceral leishmaniasis (WHO 2000). There is also a high incidence in patients with AIDS, since infection with HIV increases the risk of developing leishmaniasis by 100 to 1000 times (21), which causes increased mortality. WHO estimates that between 2% and 9% of all AIDS patients in the southern area of Europe develop visceral leishmaniasis (WHO 1995). Although there are drugs that can be used to treat the disease, the variants of the parasite in cases of visceral and cutaneous leishmaniasis that are resistant to drugs are increasingly more common, therefore it is important to develop alternative strategies for the fight against this disease, which may be the development of vaccines. The fact that patients who have recovered from a natural infection develop a strong immune response against *Leishmania* suggests the possibility of developing a vaccine against this parasite complex (19).

Different antigens have been used, conferring different levels of protection. A recent study, in which different candidates in the form of DNA were compared, has demonstrated that the gene of the LACK protein is the most promising (1). The LACK antigen (Leishmania homologue of receptors for Activated C Kinase) is a protein with a molecular weight of 36 Kda, highly conserved among the different *Leishmania* species, and which is expressed in the promastigote as well as in the amastigote. The LACK protein is a preferential target of the early anti-parasite response, as it controls the expansion of IL-4 secretory cells which lead to the disease. Studies indicate that the administration of naked DNA vectors which code the LACK antigen are capable of providing protection against *L. major,* although, despite its high immunogenicity, they do not manage to protect against murine visceral leishmaniasis (VL) when they are inoculated intradermally or intravenously (36), which could be seen as an indication that LACK may not be a useful antigen for a general vaccine against leishmaniasis based on naked DNA vectors.

However, the system used to determine the coding sequence of the antigen and the possibility of the presence of this in the cell can be a critical element that determines the antigenic efficiency, therefore LACK can be a useful antigen when its presence in the cell is determined by the inclusion of its coding sequence in a vector which may make its expression possible that may not be naked DNA. The vectors based on the Vaccinia virus have shown to be good for providing antigens for the control of infectious diseases in studies with animal models (37) and, in particular, they have demonstrated a large capacity for increasing the specific cellular immune response when the animals are administered a first immunising dose (priming) which contains different recombinant vectors (e.g. naked DNA, proteins, pseudo-particles, different viral vectors to the vaccinia virus) subsequently followed by a second booster dose with a recombinant Vaccinia virus, the vectors of the first and second dose expressing the same antigen (18, 35). The protocols which combine the recombinant Vaccinia vectors in the second dose with the administration of the recombinant vectors of naked DNA in the first dose (5, 8, 12-15, 20, 28, 30, 33) enable the expected results to be achieved in different animal models, protection being obtained which correlates with the activation of a cellular immune response, particularly the activation of CD8 T cells + IFN-γ secretors. Immunisation with DNA promotes the humoral, as well as the cellular immune response, providing protection in experimental models (34). This method offers the possibility of manipulating the immune response induced with concomitant administration of adjuvants, such as cytokines, to increase the efficiency of the vaccination (11, 16).

An example of using these strategies in which cytokines are combined with recombinant viruses derived from Vaccinia forms the Spanish patent application ES2171360, in which the use of a recombinant virus derived from a wild-type strain of Vaccinia Western Reserve (WR), that incorporates the coding sequence of the LACK protein under the control of the p7.5 promoter of the virus, tests being carried out by administering mice with different doses of this vector as well as combining it with another which contained, besides the LACK sequence, the coding sequence of the IL-12 cytokine under the control of the pE/L promoter. For its part, in the addition application to the aforementioned Spanish patent, ES2172482, the administration of the first of the recombinants to dogs is described, where it uniquely incorporates the LACK coding sequence in the second vaccination dose which forms part of the immunisation protocol where a recombinant naked DNA which equally contains the coding sequence of the LACK protein is administered in the first dose. In the first application cited, as well as in its addition, the use is proposed of recombinant vectors derived from the virulent strain (WR, Western Reserve) of the wild-type Vaccinia virus as vaccines for animals, admitting the need to develop safer vectors to enable it to be used for the vaccination of humans, proposing the MVA virus as a candidate to be used in the generation of recombinant vector vaccinations suitable for use in humans. Up until now, however, a recombinant vector analogue based on MVA with the capacity of providing protection against *Leishmania* and with a stability which would enable it to be established for use as a vaccine against leishmaniasis in humans, has still not been generated.

MVA (*Modified Vaccinia Ankara Virus*) is an attenuated form of Vaccinia derived from the Ankara strain obtained by more than 500 passes in chicken embryo fibroblasts, which has been used in around 120,000 Caucasian individuals without adverse effects (23). During the attenuation 15% of the parent genome, including genes involved in immunoregulation (4) and the range of hosts (2, 25), has been deselected (25). This virus is incapable of being replicated in cell lines and in human primary cultures (6). However, changes are not produced in the levels of expression of the viral or recombinant proteins (32). Its low virulence and its good capability in triggering cell responses (27) makes it a good candidate to be used for the generation of recombinant forms which may enable the expression of antigens against which an immune response needs to be triggered.

For this reason, the present invention has decided to choose this virus as a basis to develop a recombinant capable of expressing a coding sequence of the LACK protein of *L. infantum* or an immunogenic fragment of the same. However, unlike the recombinant vectors based on the WR strain described in application ES2171360 and in the addition of the same ES2172482, in a described realisation of the invention, the haemagglutinin (HA) locus has been chosen instead of the thymidine kinase (TK) locus for the insertion of the LACK coding sequence, as the inactivation of the haemagglutinin gene in the MVA vector makes the cell-cell fusion process easier (38), which increases the antigen presentation after an intramuscular or intradermal inoculation. Also, in the said realisation the pE/L (early/late) (39) synthetic promoter has been chosen as a promoter that regulates the expression of the sequence, instead of the promoter p7.5 which is used in the vectors described in the cited applications, which ensures the continued synthesis of the antigen during the infection process and gives rise to high production levels of the same, facilitating the generation of an immune response against the aforementioned antigen. Also unlike the previous invention, the infection with MVA produces less cell destruction than the WR wild-type strain, increasing the antigenic presentation. With all this, a vector has been obtained which gives rise to a higher immunological stimulation together with a good protection against leishmaniasis compared to the known vectors in the state of the technique used against the aforementioned disease. Moreover, unlike other vectors based on MVA known in the technique which have been attempted to be used for the vaccination of human beings against other diseases, the vector of the invention has shown to be stable and maintains the insert which it contains after subjecting it to successive passes. These characteristics and the immune response results and protection against *Leishmania* obtained in the tests carried out in the murine model demonstrate that a suitable vector to be considered for the vaccination of human beings against *Leishmania* has been obtained, particularly if it is administered in the booster dose of a vaccination protocol in which a different recombinant vector from which the same antigen can be expressed is administered in the first immune-response-triggering dose, such as naked DNA.

## DESCRIPTION OF THE INVENTION

The invention provides a recombinant MVA virus vector comprising a coding sequence of the LACK protein, characterised in that the coding sequence of the LACK protein is contained in a DNA fragment which is inserted in the haemagglutinin locus of the MVA genome between the right and left flanking regions of the haemagglutinin (HA) gene, in which DNA fragment there is an early/late pE/L synthetic promoter to which is joined the coding sequence of the LACK protein in a way that the promoter directs the expression of the said coding sequence to give rise to the LACK protein.

The LACK coding sequence present in the recombinant vector of the invention can code the complete protein or an immunogenic fragment of the same. The term "immunogenic fragment" refers to a fragment of the protein which comprises, at least, 20% or, preferably, 50% of the amino acid sequence of the protein and which is capable of triggering an immune response against the same. In a specific embodiment of the invention in which the construction is described in detail, the coding sequence present in the recombinant vector of the invention codes the complete LACK protein of *Leishmania infantum,* the cloning and characterisation of which has been described by González-Aseguinolaza *et al.* (7). The LACK gene was ceded by the coordinator of this group, Dr. Vicente Larraga of the Centro de Investigaciones Biológicas, Madrid.

The invention also refers to compositions which include at least a recombinant vector of the invention and, optionally, at least an adjuvant or an acceptable pharmaceutical vehicle. The pharmaceutically acceptable adjuvants and vehicles that can be used to form part of a composition which includes at least a recombinant vector of the invention are adjuvants and vehicles known by experts on the subject, which will be chosen depending on the administration route which is intended to be used in such a way that a composition suitable for administering by this route may be obtained. In particular embodiments of the invention, the administration route is chosen between the intraperitoneal route, the intradermal route or the intramuscular route, the intramuscular route being particularly preferred. In those cases, compositions in solution form or aqueous suspension is preferred, therefore the composition will contain a pharmaceutically acceptable diluent such as a saline solution, a saline solution buffered with phosphate (PBS) or any other pharmaceutically acceptable diluent.

The vector of the invention is safe and stable, which gives rise to a powerful cellular immune response against the LACK antigen and which, as is subsequently shown in the examples, is capable of inducing protection against leishmaniasis in the murine model. It is for this reason that another aspect of the invention establishes the use of the vector of the invention for the preparation of a drug destined to protect from leishmaniasis a mammal susceptible to developing it. When the studies were carried out to evaluate the protection generated against Leishmania infantum, the use of the vector of the invention in immunisation protocols in which a recombinant naked DNA which also expresses the LACK protein is administered in the first dose, the vector of the invention forming part of the second booster dose, gave rise to a protection similar to that observed when the virus used in the booster dose is a recombinant virus which also expresses the LACK protein, derived from the Vaccinia Western Reserve strain, while in studies in which the protection generated against *Leishmania major* is evaluated the vector of the invention gives rise to a higher protection in general to that generated by recombinant viruses derived from the Western Reserve strain and in particular superior to the already known recombinant virus in which the LACK coding sequence is inserted in the TK locus. It is for this reason that the drug can be used as protection against visceral leishmaniasis or against cutaneous leishmaniasis. The safety of the vector of the invention and the results of the protection observed in the murine model, which are considered predictive of the responses which can be observed on carrying out tests in non-human primates, make the vector of the invention a good candidate to be used in human beings for protection against leishmaniasis, although it is also an option to be administered in combination with or substituting for recombinant viruses derived from the Vaccinia Western Reserve strain which also express the LACK protein, the use of which has been proposed for the protection of other animals such as dogs.

The present application describes the vaccination methods by which the vector of the invention is administered. Although the administration of a single dose of the vector of the invention enables a cellular immune response to be observed, methods are preferred in which more than one dose, with a time gap, is administered. The fact that the virus of the invention, derived from MVA, can induce a low response against the antigens common to Vaccinia compared with recombinant viruses like those derived from the Western Reserve strain, makes its use possible in the first dose for initiating the immune response as well as in subsequent booster doses. However, heterologous vaccination methods are preferred in which the virus of the invention is administered in the second and/or subsequent booster doses, while another different recombinant vector is administered in the first dose which, preferably, also constitutes a system for the expression of the LACK protein or an immunogenic fragment of the same. Those vaccination methods are preferred in which the recombinant vector which is administered in the first dose is a naked DNA capable of expressing the LACK protein of *Leishmania infantum,* which has been shown to give rise to a good protection in the examples which are described later. A particularly preferred vaccination method is that in which the recombinant DNA vector used is the plasmid that is mentioned in the report as DNA-LACK (pCI-neo-LACK), which when combined with the vector of the invention has given rise to good results of protection against *Leishmania major* and against *Leishmania infantum,* although it is possible to use other different plasmids such as, for example, pMOK. Another possible vaccination method is that in which, in addition to the vaccination vectors, which could correspond to protein CD40L, an adjuvant is also added.

The invention will now be described in more detail by the Figures and Examples which are described below.

## SHORT DESCRIPTION OF THE FIGURES

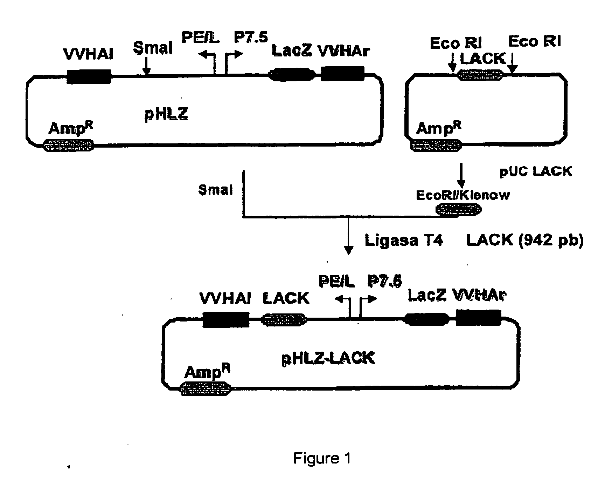

Figure 1 shows a scheme of the construction of the vector pHLZ-LACK (lower part of the figure) from pHLZ plasmids (left upper part) and pUC-LACK (right upper part).

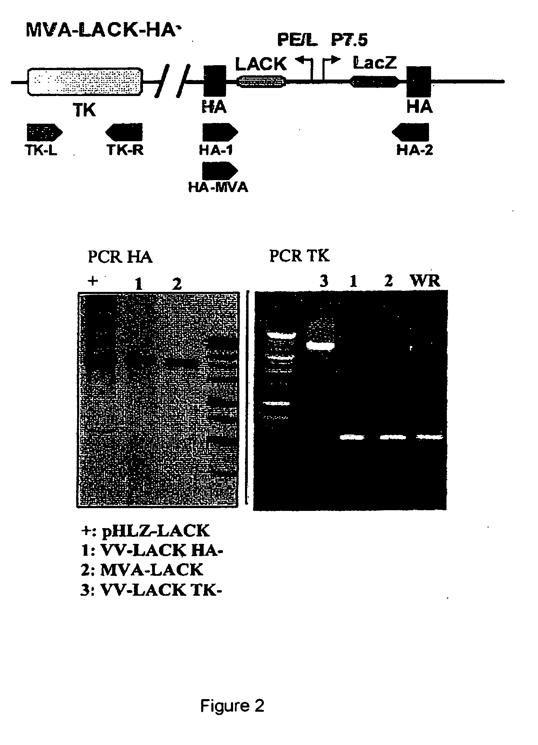

Figure 2 shows, in its upper part, a scheme of the MVA-LACK virus and the location of the areas where the oligonucleotides used are paired as primers in the PCR analysis of the HA and TK locus (for the identification of the oligonucleotides see Table 1). Photographs of the gels obtained on performing the aforementioned PCR are shown in the lower part; the left part corresponds to the analysis of the HA locus and the right part to the analysis of the TK locus. In both, the lanes correspond to the following samples: + : pHLZ LACK; 1: W-LACK HA⁻; 2: MVA-LACK; 3: W-LACK TK-; WR: Western Reserve strain.

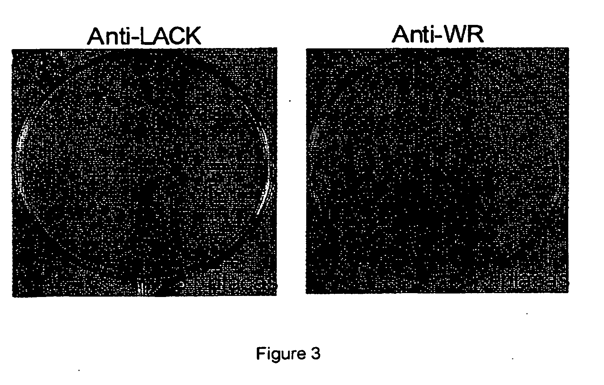

Figure 3 shows the photographs obtained after the immunostaining of the plates generated by infection of the CEF cells with the P4 stock of MVA-LACK, using an anti-LACK polyclonal antibody (photograph on the left) or anti-WR (photograph on the right).

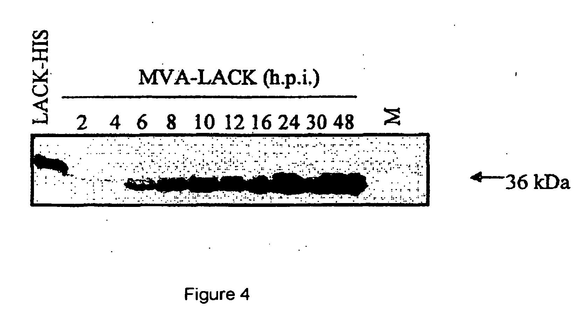

Figure 4 shows the pattern of bands obtained after reacting an anti-LACK polyclonal antibody with the bands obtained by fractionating cell extracts in an SDS-PAGE gel harvested at the post-infection times indicated over each one of the lanes. M: sample taken 48 hours after the infection had been simulated. LACK-HIS: positive control sample obtained from a strain of *E. coli* which expresses a LACK protein which has a histidine tail.

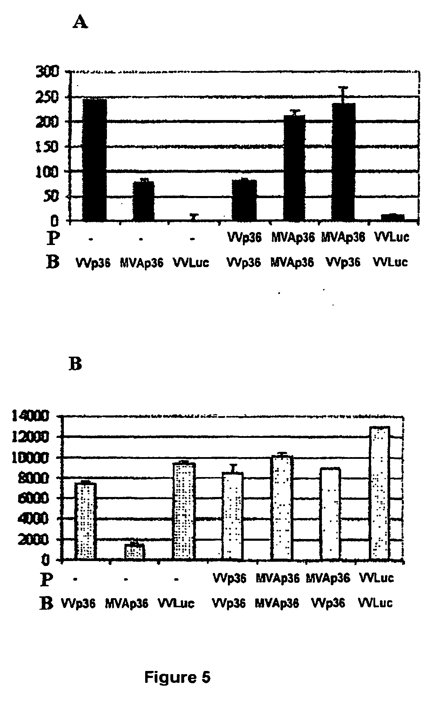

Figure 5 shows the number of secretor cells of IFN-$\gamma$ for every $10^6$ splenocytes from Balb/c mice stimulated with the vectors indicated in the abscissa and detected by using ELISPOT, which are: W p36: W-LACK HA-; MVAp36: MVA-LACK; VV-Luc: vector derived from the WR strain which contains the luciferase gene. P: first dose; B: booster dose. Part A corresponds to the secretor cells of IFN-$\gamma$ specific for the LACK protein and part B to the secretor cells specific for viral antigens.

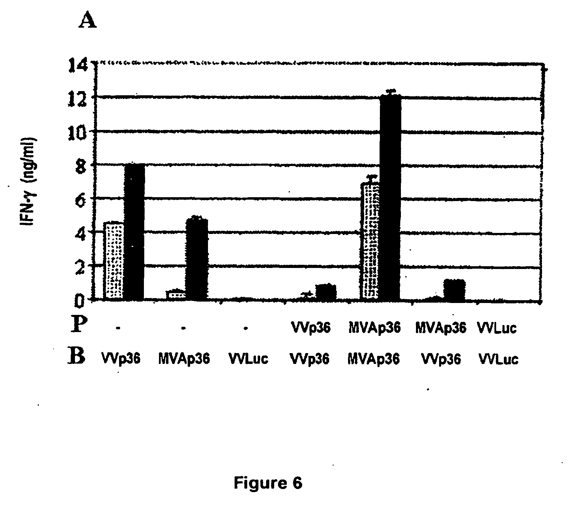

Figure 6 shows the concentration of IFN-$\gamma$, in ng/ml, detected in the supernatant of the culture of splenocytes isolated from mice inoculated with the vectors indicated on the abscissa and re-stimulated with the LACK protein (first band which appears shadowed) or with an immunostimulant class II peptide (second band, which appears filled in black). The names of the vectors correspond to: Wp36: W-LACK HA-; MVAp36: MVA-LACK; VV-Luc: vector derived from the WR strain which contains the luciferase gene. P: first dose; B: booster dose.

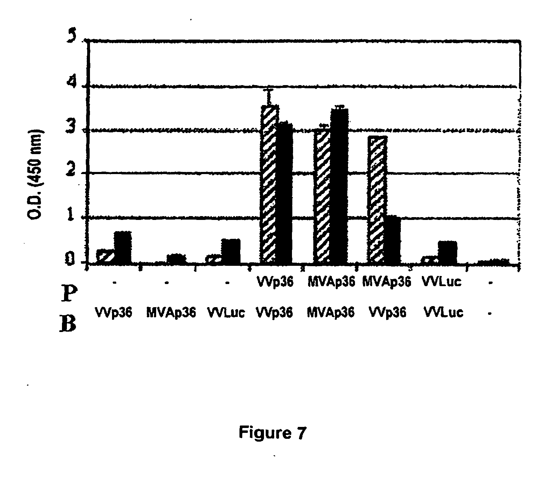

Figure 7 shows the optical density values, measured at 450 nm, obtained on carrying out the detection of antibodies specific for LACK in serum of Balb/c mice diluted 1/10, collected 8 days after the second dose of a vaccination protocol with two immunisation doses, each one of which included the vectors indicated on the abscissa, P indicating the vector inoculated in the first dose and B the one inoculated in the booster dose. The names of the vectors correspond to: Wp36: VV-LACK HA-; MVAp36: MVA-LACK; VV-Luc: vector derived from the WR strain which contains the luciferase gene. The first band of each vaccination protocol, marked with sloping lines, corresponds to the antibodies to the IgG1 isotype; the second band, filled in black, to the antibodies to the IgG2a isotype.

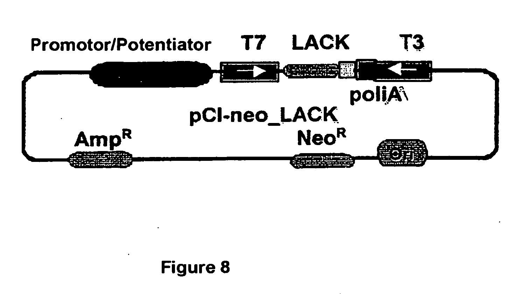

Figure 8 shows the structure of the pCI-neo-LACK (DNA-LACK) plasmid.

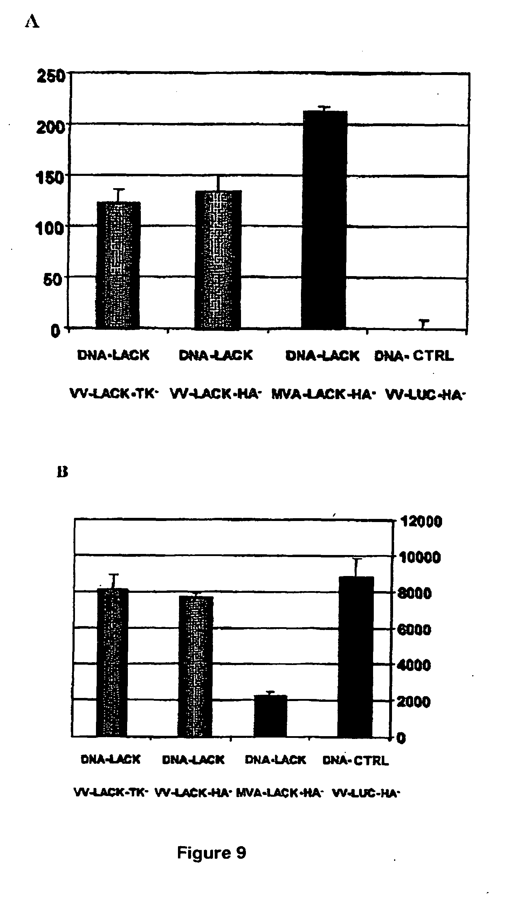

Figure 9 shows the concentration of IFN-$\gamma$ for every $10^6$ splenocytes of Balb/c mice stimulated with the vectors indicated on the abscissa, and detected using ELISPOT. P: first dose; B: booster dose. Part A corresponds to the secretor cells of IFN-$\gamma$ specific for the LACK protein and part B to the secretor cells specific for viral antigens.

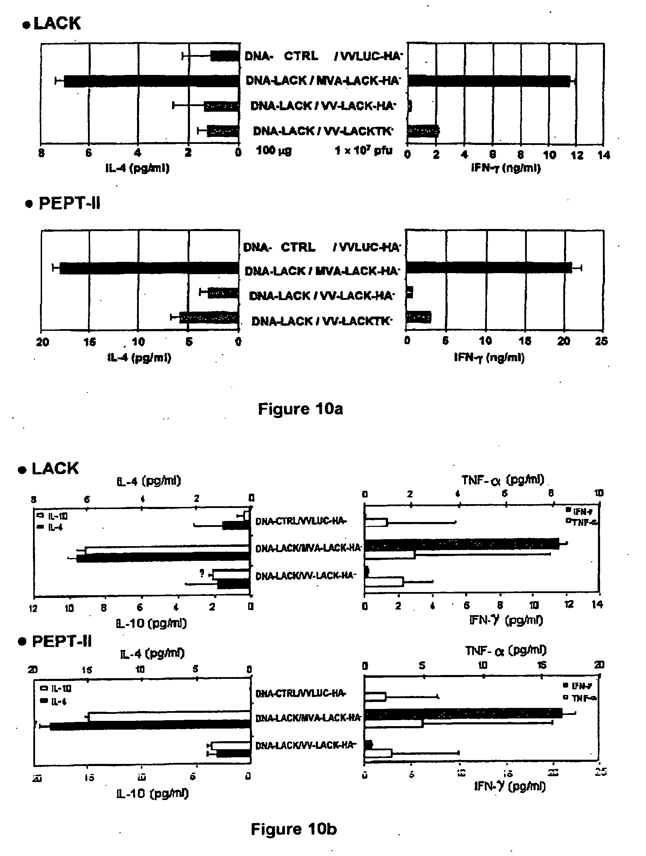

Figure 10 shows the pattern of cytokines secreted after restimulation of splenocytes isolated from Balb/c mice immunised with combinations of recombinant vectors, in which the first vector referred to is administered in the first dose and the second vector in the second dose. Both in Figure 10a and in Figure 10b, the graphs in the upper part of the figure correspond to the results obtained after restimulating splenocytes with the LACK protein, while graphs in the lower part correspond to restimulation with a class II immunodominant peptide (PEPT-II). The vector combinations used to immunize the mice are those indicated next to the bands, in which DNA-CTRL: control DNA lacking the insert with the LACK encoding sequence. In both figures, the graphs at the left show the concentration of cytokines associated with type Th2 (IL-4) responses in the case of Figure 10a and both IL-4 (shaded bands, scale on each graph) and IL-10 (empty bands, scale under each graph) in the case of Figure 10b; the graphs at the right show the concentration of cytokines associated with Th1 type response detected in each case (only IFN-$\gamma$ in the case of Figure 10a and both IFN-$\gamma$ (shaded bands, scale on each graph) and TNF-$\alpha$ (empty bands; scale on each graph) in the case of Figure 10b. The data obtained after restimulation of splenocytes obtained from mice immunised with the combination of vectors DNA-LACK/VV-LACK TK are only present in Figure 10a, and have not been included in Figure 10b.

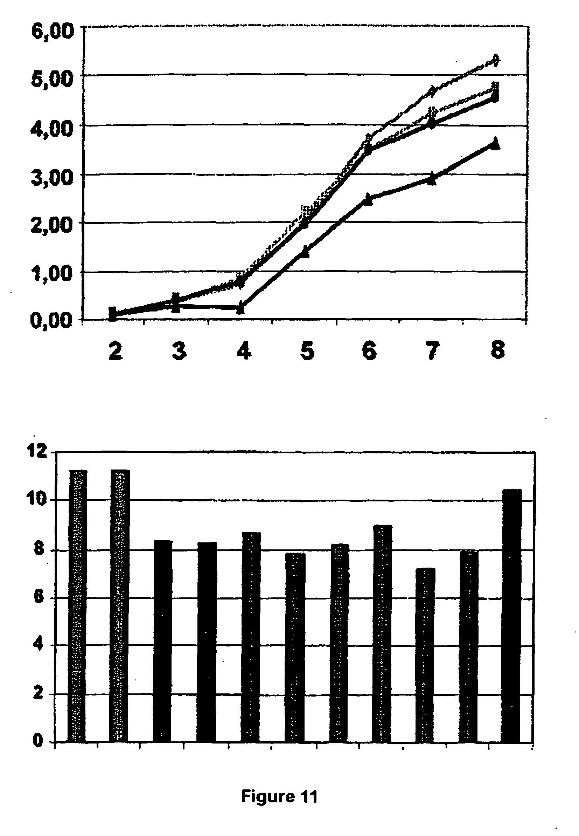

Figure 11 corresponds to the evaluation of the protection against a challenge by *L. major* to Balb/c mice immunised with different vaccination protocols. The upper part A shows a graph in which, in order, the size of the lesion is shown, expressed in millimetres, detected after the weeks that are indicated on the abscissa after the challenge with promastygotes of *L. major* to Balb/c mice immunised with DNA-LACK (100 micrograms) in the first dose and, in the second dose with 1 x $10^7$ pfu/mouse of: ($\blacklozenge$): VV-LACK TK-; ($\blacksquare$): VV-LACK HA-; ($\blacktriangle$): MVA-LACK. The points marked with the ($\bullet$) symbol correspond to the data of the mice that were administered a control DNA in the first dose and VV-Luc HA- in the second. In part B, the logarithm to the base 10 of the parasite load corresponding to the same experiment, detected in the popliteal ganglia of Balb/c mice, is shown. From left to right, band 1, corresponds to the spleen mixture of 8 mice immunised with DNA-LACK/VV-LACK-TK-; band 3; the spleen mixture of 8 mice immunised with DNA-LACK/VV-LACK-HA-; bands 3 to 10 represents a mouse spleen immunised with DNA-LACK/MVA-LACK HA-; band 11, mixture of 7 spleens from mice immunised with a DNA-control (empty plasmid) /VV-Luc HA-.

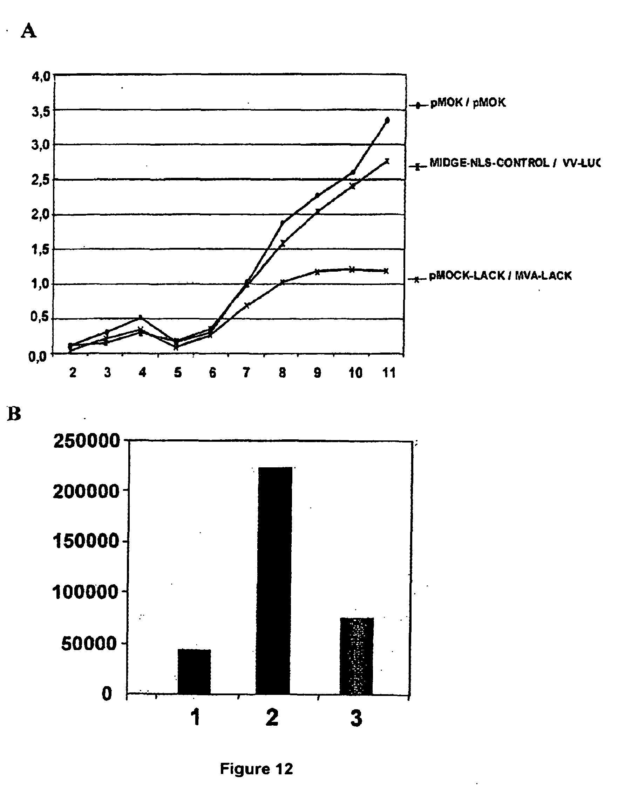

Figure 12 shows, in part A, a graph in which, on the ordinates, the size of the lesion is indicated, expressed in millimetres, detected after the weeks that are indicated on the abscissa after the challenge with promastygotes of *L. major* to Balb/c mice immunised with: pMOK in the first and in the second dose (data indicated by a circle, $\bullet$);

MIDGE-NLS in the first dose and VV-Luc in the second dose (data indicated by the symbol ✿); pMOK-LACK in the first dose and MVA-LACK in the second dose (data indicated by the symbol X). In part B of the Figure, the parasite load is indicated, expressed as the number of parasites/mg, detected in the same animals; band 1 corresponds to the immunisation with pMOK-LACK and MVA-LACK, band 2 to the immunisation with MIDGE-NLS and VV-Luc and band 3 to the immunisation with pMOK in the first and in the second dose.

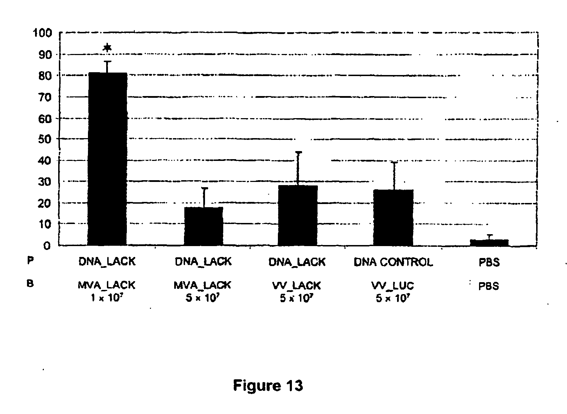

Figure 13 shows a graph in which are indicated, on the ordinates, the number of IL-2 secretor cells, specific for the LACK protein, detected for every $10^6$ splenocytes of Balb/c mice immunised with DNA-LACK in the first dose and $1 \times 10^7$ pfu/mouse of MVA-LACK in the second dose (first band); DNA-LACK in the first dose and $5 \times 10^7$ pfu/mouse of MVA-LACK in the second dose (second band); DNA-LACK in the first dose and $5 \times 10^7$ pfu/mouse of VV-LACK in the second dose (third band); Control DNA in the first dose and $5 \times 10^7$ pfu/mouse of VV-Luc in the second (fourth band); PBS in the first dose and in the second dose (fifth band).

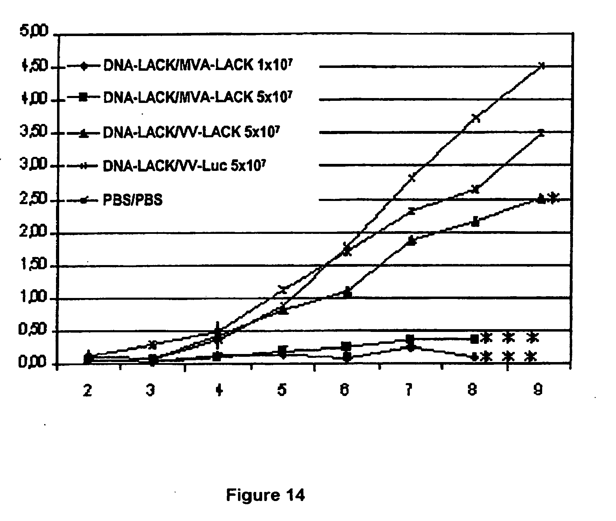

Figure 14 shows a graph in which, on the ordinates, the size of the lesion is indicated, expressed in millimetres, detected after the weeks indicated on the abscissa after the challenge with promastygotes ($5 \times 10^4$) of *L. major* to Balb/c mice immunised with DNA-LACK in the first dose and in the second dose with: (♦): $1 \times 10^7$ pfu/mouse of MVA-LACK; (■): $5 \times 10^7$ pfu/mouse of MVA-LACK; (Δ): $5 \times 10^7$ pfu/mouse of VV-LACK; (X): $5 \times 10^7$ pfu/mouse of VV-Luc. The points marked with the symbol (✿) correspond to the data from mice that were administered PBS in the first dose and in the second dose.

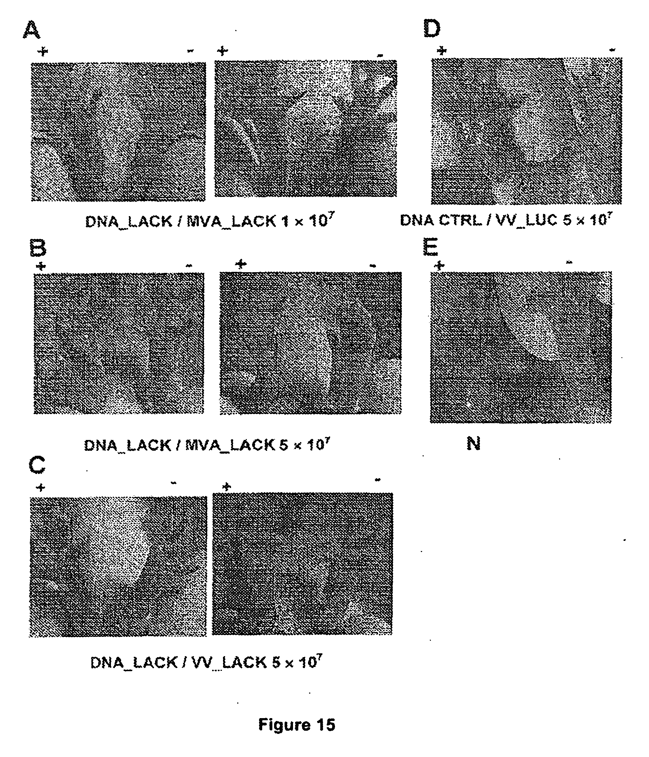

Figure 15 shows photographs taken of the mice subjected to different immunisation protocols, 8 weeks after the challenge with *Leishmania major*. The "+" signs indicate the paws in which the promastygotes ($5 \times 10^4$) were inoculated whilst the "-" signs correspond to the paws where promastygotes were not inoculated, which served as controls. The immunisations were carried out with: Panel A: DNA-LACK in the first dose and $1 \times 10^7$ pfu/mouse of MVA-LACK in the second dose; panel B: DNA-LACK in the first dose and $5 \times 10^7$ pfu/mouse of MVA-LACK in the second dose; panel C: DNA-LACK in the first dose and $5 \times 10^7$ pfu/mouse of VV-LACK in the second dose; panel D: Control DNA in the first dose and $5 \times 10^7$ pfu/mouse of VV-Luc in the second dose; panel E: PBS in the first dose and in the second dose (group N: *naive*).

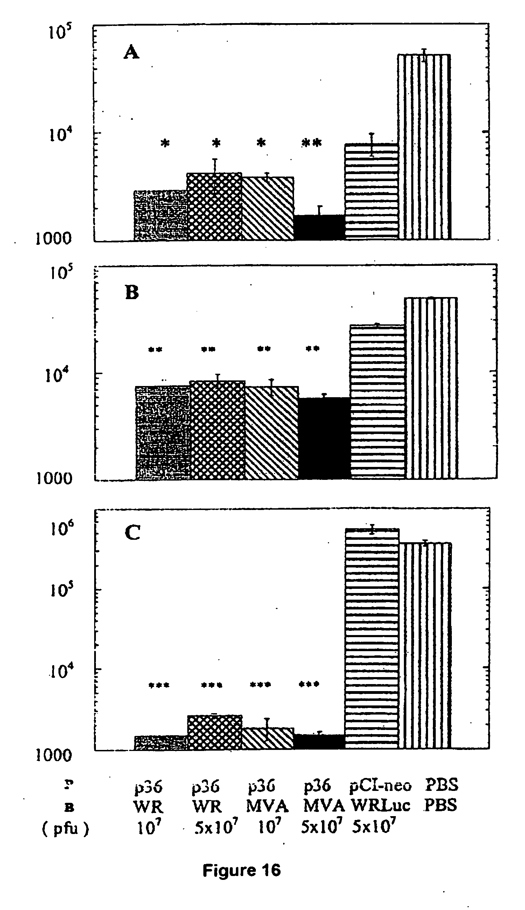

Figure 16 shows graphs which represent the parasite loads detected in different organs of Balb/c mice, immunised with different treatments, detected one month after an intradermal challenge with $1 \times 10^7$ metacyclic promastygotes of *L. infantum.* Graph A: load detected in spleen; graph B: load detected in liver; graph C: load detected in lymphatic drainage nodule. In each one of them, the bands correspond to the following vaccination protocols: first band (shaded), DNA-LACK in the first dose and $1 \times 10^7$ pfu/mouse of VV-LACK in the second dose; second band (with reticle), DNA-LACK in the first dose and $5 \times 10^7$ pfu/mouse of VV-LACK in the second dose; third band (with sloped lines), DNA-LACK in the first dose and $1 \times 10^7$ pfu/mouse of MVA-LACK in the second dose; fourth band, DNA-LACK in the first dose and $5 \times 10^7$ pfu/mouse of MVA-LACK in the second dose; fifth band (filled in black), pCI-neo plasmid in the first dose and $5 \times 10^7$ pfu/mouse of VV-Luc in the second dose; last band (with vertical lines), PBS in both doses. The asterisks on the bands indicate there are statistically significant differences between the data: ***: p<.001; **: p<.01; *: p<.05.

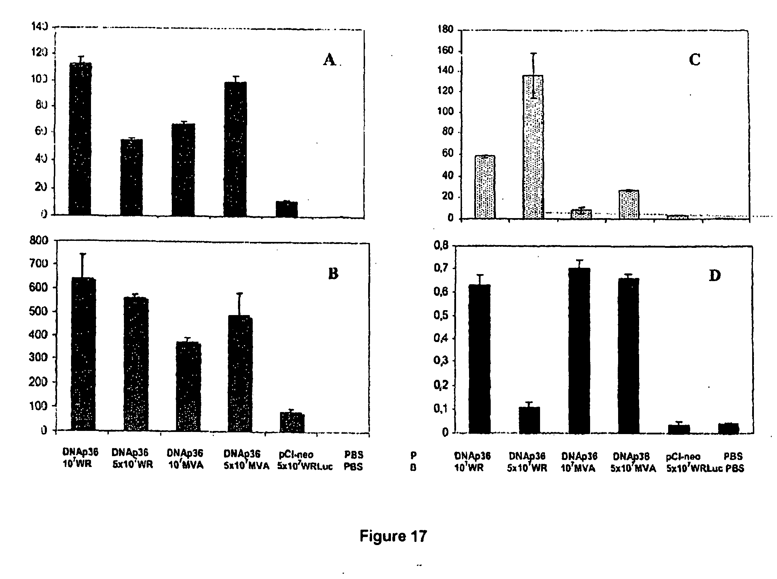

Figure 17 shows graphs which correspond to the immune response detected in the spleens of Balb/c mice subjected to different immunisation treatments, to those who had not been inoculated with *L. infantum.* Graph A corresponds to the levels of IFN-γ, in ng/ml, detected in the supernatant of cultures of splenocytes re-stimulated with the LACK protein. Graph B corresponds to the number of IFN-γ producer cells specific for LACK, detected using ELISPOT, per $10^6$ splenocytes. Graph C corresponds to the levels of TNFα/LT, in ng/ml, detected in the supernatant of cultures of splenocytes re-stimulated with the LACK protein. In each of the graphs, the bands correspond to the following vaccination protocols: first band, DNA-LACK in the first dose and $1 \times 10^7$ pfu/mouse of VV-LACK in the second dose; second band, DNA-LACK in the first dose and $5 \times 10^7$ pfu/mouse of VV-LACK in the second dose; third band, DNA-LACK in the first dose and $1 \times 10^7$ pfu/mouse of MVA-LACK in the second dose; fourth band, DNA-LACK in the first dose and $5 \times 10^7$ pfu/mouse of MVA-LACK in the second dose; fifth band, pCI-neo plasmid in the first dose and VV-Luc in the second dose; last band, PBS in both doses.

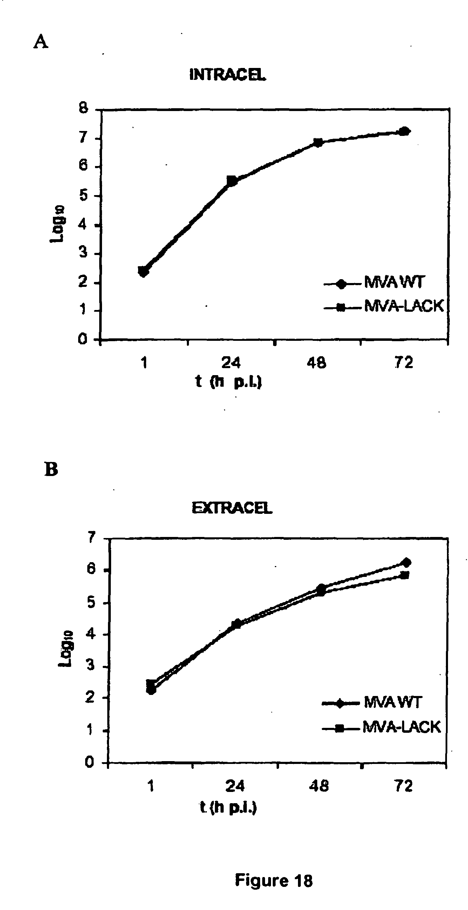

Figure 18 shows both graphs, relating to the growth efficiency of the recombinant virus MVA-LACK, graphs which represent, on the ordinates, the logarithm of the concentration of the MVA-LACK virus (data represented by shaded squares: ■) and of the wild type virus MVA-WT (data represented by ♦) detected or associated with cells (Intracell: intracellular, upper graph: A) or in the growth medium (Extracell: extracellular, lower graph B) by immunostaining carried out in BHK-21 cells, after times (t) post-infection indicated in hours (h) on the abscissa.

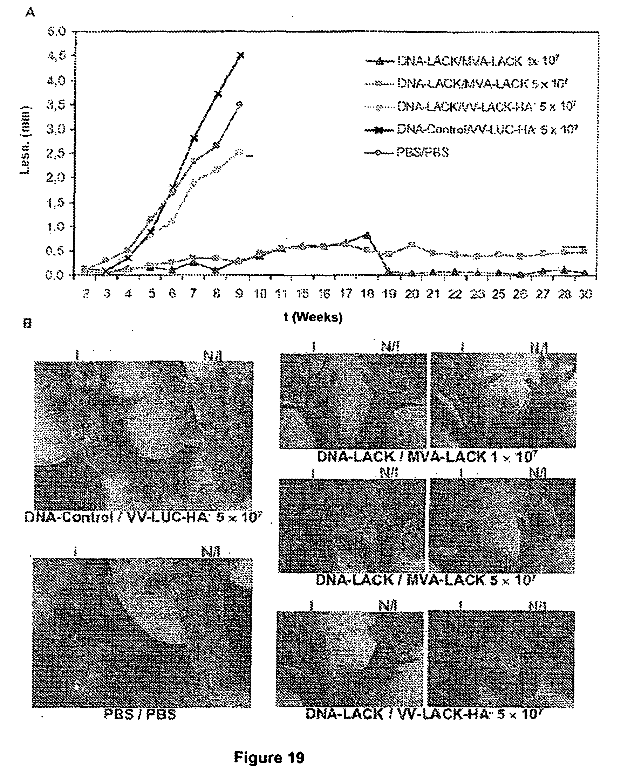

Figure 19 shows the lesions developed by mice inoculated with *L. major* promastygotes, in relation to the previous immunisation treatment. The higher part of the Figure, A, depicts a graph in which the ordinates represent the size of the lesion (Lesn), in millimetres observed in mice (mm), after a time, expressed in weeks, indicated on the abscissa, from the moment when the mice were inoculated with *L. major* promastygotes; the combinations of immunisation vectors administered to each group of mice previously inoculated with *L. major* are indicated on the graph. The lower part, B, shows photographs of the legs of mice submitted to different immunisation protocols, 9 weeks after challenge with *Leishmania major,* photographs labelled with the letter "I" indicate the legs in which the promastygotes were inoculated ($5 \times 10^4$), while the letters N/I indicate those used as control, in which no promastygotes were inoculated. The abbreviations given to the different immunisation groups correspond to: DNA-LACK/MVA-LACK $1\_10^7$ : DNA-LACK in the first dose and $5 \times 10^7$ ufp/mouse of MVA-LACK in the second dose; DNA-LACK/MVA-

LACK 5_10[7]: DNA-LACK in the first dose and 5 X 10[7] ufp/mouse of MVA-LACK in the second dose; DNA-LACK/VV-LACK-HA:DNA-LACK in the first dose and 5 X 10[7] ufp/mouse of VV-LACK in the second dose; DNA-Control/VV-LUC-HA: control plasmid lacking LACK encoding insert in the first dose and 5 X 10[7] ufp/mouse of VV-Luc in the second dose; PBS/PBS: PBS both in the first and the second dose.

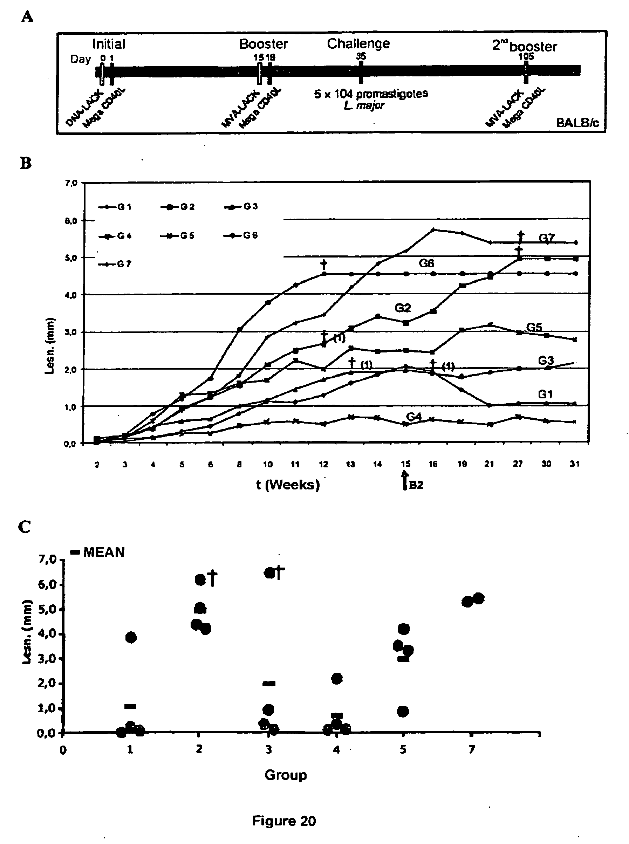

Figure 20 corresponds to the assay to study the increased protection conferred by administering an adjuvant, CD40L, to Balb/c mice submitted to different immunisation treatments and to a challenge of *L. major* promastygotes. The upper part, A, shows a schematic representation of the immunisation protocol followed. The intermediate part, B, shows a graph representing on the ordinates the size of the lesion (lesn), in millimetres (mm) observed in the mice, after a given time period, expressed in weeks, indicated on the abscissa, from the time the mice were inoculated promastygotes of *L. mayor,* the abbreviations G1 to G7 for each line of data indicate the assay group referred to (Group 1 to Group 7), the signs † indicate the times when the mice were sacrificed and the arrow marked as B2 indicate the times when mice from groups 1,3 and 4 received their third dose of immunisation vector (2nd booster dose "booster 2"). In the lower part of the Figure, labelled C, the sizes of the lesions are shown in millimetres, observed in each of the surviving mice from groups 1,2,3,4,5 and 7, 27 weeks after the challenge; each circle represents a mouse, and the height at which this circle is found corresponds to the size of the lesion observed, and the group it belongs to is indicated by the reading on the abscissa. The signs † together with a circle from part C indicate that the mouse was sacrificed before 27 weeks. The groups correspond to the following immunisation combinations: Group 1 (G1): DNA-LACK/MVA-LACK/MVA-LACK; Group 2 (G2): DNA-LACK + MegaCD40L($\mu$g) /MVA-LACK + MegaCD40L (1$\mu$g); Group 3 (G3) DNA-LACK + MegaCD40L(10$\mu$g)/MVA-LACK + MegaCD40L (10$\mu$g)/MVA-LACK; Group 4 (G4) DNA-LACK + MegaCD40L(20$\mu$g)/MVA-LACK + MegaCD40L (20$\mu$g)/ MVA-LACK + MegaCD40L (20$\mu$g); Group 5 (G5): DNA-Control/MVA-WT; Group 6 (G6): PBS/PBS ; Group 7 (G7): PBS + MegaCD40L (10$\mu$g)/PBS + MegaCD40L (10$\mu$g).

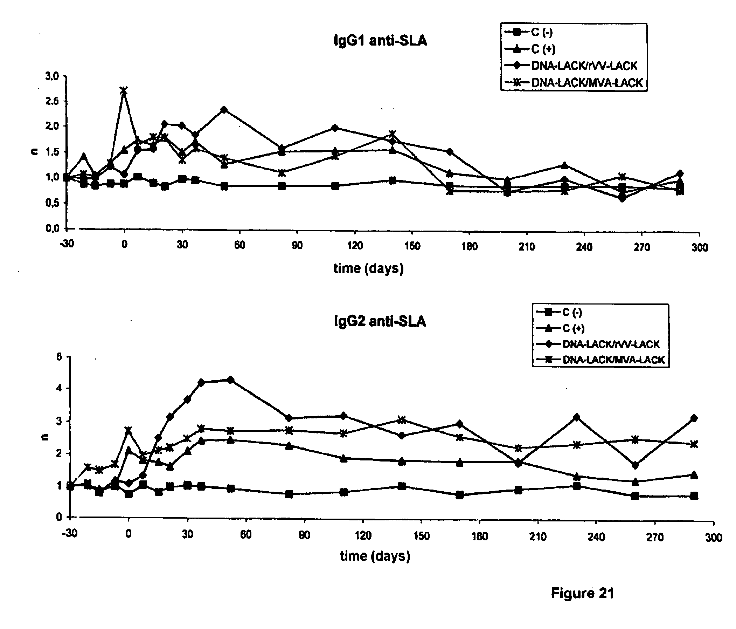

The graphs depicted in Figure 21 represent the evolution over time of the concentration of anti-SLA antibodies (soluble Leishmania antigen) IgG1 type (upper part, A) or IgG2 type (upper part, B) in Beagle dogs inoculated with promastygotes of *Leishmania infantum.* This is done by indicating on each graph, on the ordinate axis, the absorbance value corresponding to transformation of the substrate OPD produced by plasma samples from dogs submitted to ELISA, N, the value expressed as per 1 relative to the value obtained on day 30, the day the sample was taken is indicated on the abscissa, with day 0 corresponding to the day of inoculation with the promastygotes. Each curve corresponds to a different previous immunisation group: shaded square■ : Negative control (C(-)); shaded triangles ▲: Positive control (C(+)); shaded rhombi ♦: the group immunised with DNA-LACK/rVV-LACK before the challenge with promastygotes; six point asterisk (*): the group immunised with DNA-LACK/MVA-LACK before challenge with promastygotes.

## EXAMPLES

### Example 1.- Construction and characterisation of the recombinant MVA-LACK virus.

#### 1.1.- Construction of the pHLZ-LACK plasmid vector

For the construction of a recombinant MVA virus which contains a coding sequence of the LACK protein of *L. infantum,* an intermediate construction pass of a plasmid, pHLZ- LACK, was necessary, which enabled the insertion of the coding sequence of the LACK protein in the HA locus of the MVA genome. This plasmid contains regions flanking left and right of the haemagglutinin gene (HA), and the gene resistant to ampicillin. Between the flanking regions of the HA gene, there are two Vaccinia promoters in the opposite direction: the viral p7.5 promoter which directs the expression of the $\beta$-gal gene, and the early/late (pE/L) synthetic promoter, which directs the expression the gene of the LACK protein of *L. infantum.* For its construction another two plasmids were used:

- **pUC LACK**: a plasmid derived form the pUC19 vector which contains the gene of the LACK protein of *L. infantum* in the position of the EcoRI cut, besides the elements required for the replication and selection in *E. coli.* This vector was generated by Dr. Gloria González-Aseguinolaza from a mould of genomic DNA of *L. infantum* using PCR. The PCR product has a length of 942 base pairs (bp) and the codifying region 312 amino acids (7).
- **pHLZ:** (7400 bp). A plasmid generated in the laboratory of the inventors which enables the insertion of genes into the Vaccinia genome by homologous recombination in the encoding region of HA. After the addition of the $\beta$-galactosidase substrate, X-gal, the development of blue plaques enables the recombinant viruses to be selected.

The pHLZ-LACK vector is generated from these two plasmids in the following way:

A 942 bp fragment of DNA which contains the coding sequence of the LACK protein of *L. infantum* is purified from the pUC LACK plasmid. For this, the pUC LACK vector is directed with the *EcoRI* restriction enzyme, and the fragment corresponding to the LACK gene is purified in agar gels. The ends of the aforementioned fragment were made blunt by treatment with *Klenow* and is cloned in the plasmid by insertion into pHLZ Vaccinia (previously directed with *SmaI* and dephosphorylated by incubation with alkaline phosphatase (CIP)), thus generating the pHLZ-LACK (8342 bp) insertion vector. The selection of the recombinant plasmids is carried out by analysing the β-gal activity.

The construction of this pHLZ-LACK insertion vector is shown schematically in Figure 1.

1.2. Construction of the MVA-LACK recombinant virus

Primary cells from chicken embryos (CEF: chicken embryo fibroblast), obtained from 11 days old SPF eggs (INTERVET), were infected with MVA (specifically MVA-F6, pass 586, provided by Gerd Sutter) to an infection multiplicity of 0.05 pfu/cell. After 1 hour of absorption, the cells were transfected with 5 μg of DNA of the pHLZ-LACK plasmid using lipofectamine in accordance with the instructions of the manufacturer (INVITROGEN). 72 hours post-infection the cells were collected into 1 ml of medium, three freeze/thaw cycles were carried out, were sonicated and used for the selection of recombinant viruses.
The recombinant viruses that contained the LACK gene of *L. infantum* and the β-gal gene were selected by consecutive purification passes in CEF cells and stained with 5-bromo-4-chloro-3-indolyl-β-galactosidase (300 μg/ml). After 6 purifying cycles the purified recombinant virus was obtained, without contamination of the wild MVA virus. The recombinant, named 3.111.2.1.1.2 (MVA pass 592) was used to prepare a second stock, P2, with a titration of $3 \times 10^7$ pfu/ml. The P3 stock was prepared from the CEF cells infected to a multiplicity of 0.05 pfu/cell and was purified through two 36% saccharose matrices. The titration of this stock is $0.975 \times 10^9$ pfu/ml. The sequencing of the insert present in its genome gave rise to the nucleotide sequence which is shown in SEQ ID NO:6.

1.3 Characterisation of the MVA-LACK virus

To confirm the homogeneity and integrity of the genes included in the MVA-LACK recombinant virus, an analysis using PCR was carried out. The viral DNA was purified from CEF cells infected by the MVA LACK virus (stock P2) to an infection multiplicity of 5 pfu/cell. After verifying the integrity of the DNA by analysis in agar gel, PCR analysis was carried out for the haemagglutinin and thymidine kinase locus, using the oligonucleotides shown in Table 1 as primers, and their location in relation to the haemagglutinin and thymidine kinase locus are shown in the upper part of Figure 2.

Table 1.- PCR primers by analysis of the HA and TK locus

| Oligonucleotides | Sequence | |
|---|---|---|
| TK-L | 5' TGATTAGTTTGATGCGATTC 3' | (SEQ ID NO: 1 |
| TK-R | 5' TGTCCTTGATACGGCAG 3' | (SEQ ID NO: 2) |
| HA-1 | 5' GTCACGTGTTACCACGCA 3' | (SEQ ID NO: 3) |
| HA-2 | 5' GATCCGCATCATCGGTGG 3' | (SEQ ID NO: 4) |
| HA-MVA | 5' TGACACGATTACCAATAC 3' | (SEQ ID NO: 5) |

To carry out the analysis, for comparison purposes, the DNA extracted from CEF cells infected with the Western Reserve (WR) wild strain was used as a negative control and the pHLZ-LACK plasmid as a positive control. The VV-LACK HA⁻ recombinant virus was also included in the analysis, which was prepared in a form analogous to MVA-LACK and which contained the same LACK coding sequence equally inserted in the HA locus although, unlike MVA-LACK, the Western Reserve strain of Vaccinia, competent for replication, was used for its construction. Throughout the present report, whenever the VV-LACK virus is mentioned, it will refer to this recombinant virus, calling it VV-LACK-HA⁻ in those cases where it is required to distinguish it from the VV-LACK-TK⁻ virus, in which the LACK sequence is inserted in the thymidine kinase locus. In this and the following examples, and also in the Figure legends and in their descriptions, the middle or low hyphen used in the names of vectors is indiscriminate and does not signify any difference between them, such that "MVA-LACK" and "MVA_LACK" correspond to the same vector. The same occurs with the pairs "VV_LACK HA"/"VV-LACK HA", "VV_LACK TK/VV-LACK TK" and "VV_LACK"/"VV-LACK". The absence or presence of the hyphen between abbreviations that compose the vector name should not be interpreted either as indicating any difference; hence VV-Luc and WLuc refer to the same vector, which are also referred to in some parts of the report, in a more informative manner, as VV-Luc HA-.
The photographs of the gels obtained are shown in the lower part of Figure 2. In the left part of this same Figure, which corresponds to the PCR analysis of the haemagglutinin locus, it is observed that the MVA-LACK (lane 2) as well as the VV-LACK-HA⁻, give rise to bands which are located at the height of the pHLZ-LACK positive control, which is compatible with the presence of the complete insert integrated into the HA position. In the right part, corresponding to the PCR analysis of the TK locus, it is verified that MVA-LACK (lane 2) as well as VV-LACK-HA⁻ (lane 1) give rise to bands of identical size to those generated from the DNA extracted from cells infected with WR wild strain, while the VV-LACK-TK⁻ vector (lane 3), which contains the same sequence but inserted in the TK *locus*, gives rise to a much bigger band.

## Example 2.- Stability of MVA-LACK

To verify that MVA LACK can be amplified without losing the expression of the exogenous gene, a stability test was carried out. The recombinant virus was amplified from the P2 to the P4 stock in CEF cells to a multiplicity of infection of 0.05 pfu/cell. The plaques generated with the P4 stock were analysed by immunomarking with an anti-WR and anti-LACK polyclonal antibody. The results, shown in Figure 3, demonstrated that 100% of the plaques recognised by the anti-WR antibody, were also positive for LACK. The LACK gene, therefore, remained in stable form in the recombinant MVA-LACK virus.

## Example 3.- Kinetics of LACK protein expression

To evaluate the expression of the LACK protein with time, single layers of BHK-21 cells were infected with 5 pfu/cell of MVA-LACK. The cell extracts were harvested at different times post-infection, were fractionated in SDS-PAGE gels, were transferred to nitrocellulose membranes and were reacted against an anti-LACK polyclonal antibody (generated in the laboratory of the inventors by immunising rabbits with purified LACK protein). The results obtained are shown in Figure 4. In this Figure it can be seen that it detected high levels of the LACK antigen from 4 hours after the infection, its production accumulating with time up to 48 hours.

## Example 4.- Immunogenicity of MVA-LACK

To evaluate the immune response induced by MVA-LACK in comparison with that induced by VV-LACK, female mice of the susceptible Balb/C species (n=4) were immunised with one ($1 \times 10^7$ pfu/mouse) or two ($5 \times 10^7$ pfu/mouse) doses of VV-LACK HA⁻; MVA-LACK or VV-Luc as control. 8 days after the second immunisation, the mice were sacrificed and the spleen were processed using the ELISPOT technique. Two assays were carried out using the ELISPOT technique, (which has been described previously (33), to evaluate the number of IFN-γ secretor cells: in the first, shown in part A of Figure 5, the number of IFN-γ secretor cells specific for LACK was detected; in the second, shown in part B of Figure 5, the number of CD8+ cells, IFN-γ secretor cells specific for the viral antigens pertaining to MVA and W vectors themselves.

Additionally a study was carried out, in which the splenocytes isolated from the inoculated animals were cultured and re-stimulated with the LACK protein, later the quantity of IFN-γ secreted by the splenocytes in the supernatant of the cultures being detected using ELISA. The results obtained are shown in Figure 6.

The results of the ELISPOT show that the number of CD8+ IFN-γ secretor cells specific for LACK is 3.15 times less in the animals inoculated with MVA-LACK compared to those who received VV-LACK when the received a single dose. In keeping with this, the quantity of IFN-γ secreted by the splenocytes re-stimulated with the protein was 5 times less. However, the number of CD8+ IFN-γ secretor cells specific for the viral antigens was 5 times higher in the animals inoculated with VV-LACK. The fact is that the immune response induced against the vector in the case of MVA produces a booster effect after a second immunisation dose. Two doses of VV-LACK dramatically decreases the number of CD8+ IFN-γ secretor cells: however, when the animals received MVA-LACK in the first dose and MVA-LACK or VV-LACK in the second booster dose, both one or the other recombinant vectors were capable of amplifying the immune response. Although the number of CD8+ IFN-γ secretor cells was similar in both cases, the quantity of IFN-γ secreted and measured by ELISA was 70 times higher when the booster dose is carried out with MVA-LACK and 86 times higher when a single dose of MVA-LACK is received.

Next, the pattern of the antibodies generated after immunisation is evaluated. Different types of IgG isotypes were considered as indicators of the response against *L. infantum.* IgG2a is associated with asymptomatic conditions while the IgG1 isotype is related to disease. 8 days after the second immunisation serum was collected from the animals and it was evaluated for the presence of antibodies specific for LACK and its isotypes. The results obtained in a dilution of 1/10 of the sera are shown in Figure 7. In them, it can be seen that the highest levels of IgG2a (associated with a Th1 response) were found after two doses with MVA-LACK.

Taken together, the results obtained demonstrate that two doses of MVA-LACK induce a strong Th1 type response against LACK antigen.

**Example 5.- <u>Immune response generated in heterologous immunisation protocols with naked DNA in the first dose and MVA-LACK in the booster dose.</u>**

The immune response generated by the MVA-LACK virus was also evaluated in a heterologous immunisation protocol based on a first immunisation dose with a recombinant vector of naked DNA which enables the expression of the LACK protein in mammal cells and a second dose with recombinant viruses derived from Vaccinia. For this, pCI-neo-LACK plasmid was used in the first immunisations, which hereinafter will be referred to as DNA-LACK, which is an expression vector in mammal cells which like MVA-LACK expresses the LACK protein of *L. infantum.* This plasmid was generated by insertion of the LACK gene in the Smal location of pCI-neo, and it differs from that patented by the Consejo Superior de Investigaciones Cientificas (Higher Council of Scientific Research) (CSIC) as a DNA-LACK vector in the LACK insertion site, as the vector of the CSIC was cloned in the EcoRI/XbaI location of pCI-neo. It contains the cytomegalovirus promoter and the genes resistant to ampicillin and neomycin, arranged as shown in Figure 8.

Six to eight weeks old female Balb/c mice were inoculated intradermally with the DNA plasmid vector, DNA-LACK. 15 days later, a second inoculation, by the intraperitoneal route, was carried out with $1 \times 10^7$ pfu/mouse of MVA-LACK (stock P3). VV-LACK TK⁻ (previously used and generated in the laboratory), and which has been shown to confer a certain degree of protection when it is used at a dose of $5 \times 10^7$ pfu/mouse (9), VV-LACK HA- and VV-Luc, were inoculated as controls. Three weeks after the last immunisation, the animals were sacrificed and the spleens were processed using the ELISPOT technique. An ELISPOT was carried out to evaluate the number IFN-γ secretor cells specific for the LACK protein as well as those specific for Vaccinia antigens.

The results obtained are shown in Figure 9. In the part A of this Figure it can be seen that the group immunised with MVA-LACK in the second dose (third band of each one of the graphs) demonstrated development of a higher cellular immune response against LACK than the viruses based on the WR wild strain. Part B of the Figure shows that the anti-viral response was much less in that same group immunised with MVA-LACK, as was expected on being an attenuated vector.

As the ratio of the immune response between Th1 types (associated with protection) and Th2 types (associated with susceptibility) is critical for the control of leishmaniasis, the type of cellular response induced was evaluated after the immunisation described earlier. For this, the splenocytes isolated from the immunised mice were re-stimulated with the LACK protein (upper part of Figure 10) or with a class II immunodominant peptide of the same (lower part of Figure 10). After 72 hours of re-stimulation, the supernatants were harvested and the presence of type Th1 (IFN-y) or Th2 (IL-4) cytokines in these supernatants was determined using ELISA. The graphs of Figure 10 show that the splenocytes produced more IFN-γ and IL-4 than the rest of the groups. Despite the higher levels of IL-4, the Th1/Th2 ratio, measured indirectly as the quantity of IFN-γ/IL-4, was higher in the group that received MVA-LACK in the second dose (IFN-γ/IL-4 = 1641 in the case of re-stimulation with the LACK protein and IFN-γ/IL-4 = 1163 in the case of the class II peptide), indicating development of a Th1-type immune response.

These results are confirmed when they are plotted together with data for TNF-α (associated with type Th1 responses such as IFN-γ) and those relating to IL-10 (associated with type Th2 responses such as IL-4), as shown in Figure 10b. The highest levels of IFN-γ are detected in the mice (groups of mice immunized with DNA-LACK in the first dose and MVA-LACK in the second) in which the highest levels of TNF-α are also detected (35.9 pg/ml) when restimulated with LACK and 63.3 pg/ml when restimulated with peptide), data clearly higher than those corresponding to the group immunised with DNA LACK/VV-LACK-HA (where 18 pg/ml are detected when restimulated with LACK and 25 pg/ml when restimulated with peptide) and the control groups (where 9 pg/ml and 18 pg/ml are detected, respectively). With respect to the Th2 type cytokines, inclusion of data for IL-10 are in agreement with the highest levels detected in the groups immunized with DNA-LACK/MVA-LACK. The Th1:Th2 ratio corresponded to groups receiving MVA-LACK in the second dose, independently of IL-4 and IL-10 levels, confirming a clear tendency of the immune response towards Th1 type in this group.

The next step was to determine what population of T-cells (CD4+ or CD8+) was involved in the secretion of Th1-type cytokines (IFN-γ and TFN-α). With this aim, animals were immunised with immunisation regimes analogous to those used to obtain the results shown in Figure 8, that is, a first response priming dose with DNA-LACK and a second booster dose with MVA-LACK (dose of $1 \times 10^7$ pfu or $5 \times 10^7$ pfu) or VV-LACK-HA⁻ (dose of $1 \times 10^7$ pfu or $5 \times 10^7$ pfu), while the animals used as controls received DNA-CONTROL (the pCI-neo plasmid vector, without the LACK sequence) in the first response priming dose and VV-Luc-HA- in the second booster dose. 14 days after the administration of the booster dose, the spleens and splenocytes were collected and were re-stimulated with LACK protein or with RPMI as a control. During the last 5 hours of re-stimulation a new stimulation together with Brefeldin A was added. Next, the intracellular cytokines were stained and analysed by flow cytometry. The results are shown below in Table 2.

**Table 2.- Cell types detected by flow cytometry after staining cytokinase cells following the immunisation protocol**

| INITIAL DOSE | BOOSTER DOSE | IFN-γ CD8+ PRODUCING CELLS | TNF-α CD8+ PRODUCING CELLS | IFN-γ CD4+ PRODUCING CELLS | TNF-α CD4+ PRODUCING CELLS |
|---|---|---|---|---|---|
| DNA-LACK | VV-LACK 1 X 10$^7$ | 10.1 | 10.4 | 1.8 | 1.6 |
| DNA-LACK | VV-LACK 5 X 10$^7$ | 8.3 | 7.5 | 1.9 | 1.9 |
| DNA-LACK | MVA-LACK 1 X 10$^7$ | 23.4 | 15.7 | 2.9 | 1.9 |
| DNA-LACK | MVA-LACK 5 X 10$^7$ | 12.7 | 11.2 | 2.5 | 1.9 |
| DNA CONTROL | VV-Luc 5 X 10$^7$ | 1.7 | 1.5 | 0.7 | 1.0 |
| PBS | PBS | 1.5 | 1.2 | 0.3 | 0.4 |

The data in Table 2 demonstrates that the cellular immune response is principally due to the CD8+ population. As a conclusion, immunisation based on DNA-LACK/MVA-LACK confers higher percentages of CD4+ and CD8+ specific cell secretors of IFN-γ and TNF-*a*.

**Example 6.- Tests of protection against *Leishmania major***

6.1. Comparison of the protection generated by inoculation of different recombinant vectors derived from Vaccinia that express LACK in the booster dose

Having established that immunisation with MVA-LACK is a powerful inducer of a cellular immune response and this being associated with protection against leishmaniasis, a test of the protection against *Leishmania major* was then carried out using a heterologous immunisation protocol based on DNA-LACK/MVA-LACK. 6 weeks old female Balb/c mice were inoculated intradermally with 100 μg of the DNA plasmid vector that expresses the LACK protein, DNA-LACK or with empty DNA (without insert) as control. 15 days later, a second inoculation, by the intraperitoneal route, was carried out with 1 x 10$^7$ pfu/mouse of MVA-LACK, VV-LACK TK-, VV-LACK HA- or VV-Luc. Three weeks after the last immunisation, the animals were challenged with 5 x 10$^4$ promastygotes of *L. Major* subcutaneously in the pad of the paw. Lesions at the site of the inoculation and the parasite load in the ganglia that drain the foot pad (popliteal ganglia) measured 7 weeks after the challenge, were considered as protection parameters.

The results obtained by representing the evolution of the size of the lesion with time are shown in the upper part of Figure 11. In this, it can be observed that the group immunised with DNA-LACK/MVA-LACK had a reduction of 20% compared with the groups immunised with DNA-LACK and one of the VV-LACK vectors.

To confirm that the reduction in the size of the lesion is correlated with protection, and not only with inflammation, the parasite load was measured in the popliteal ganglia, obtaining results which are represented in the lower part of Figure 11. The mice that received DNA-LACK/MVA-LACK had a greater reduction in parasite load (up to 1000 times) than the groups immunised with DNA-LACK/VV-LACK.

6.2. Protection generated using a different DNA vector in the first dose

Another experiment was carried out to evaluate protection against *L. Major*. The animals were immunised with a DNA vector that expresses a different LACK protein of DNA-LACK, pMOK-LACK, and as controls, the minimalistic MIDGE vector (minimalistic, immunologically defined gene expression) MIDGE-NLS (MOLGEN®) or an empty vector without a pMOK insert. 15 days later, the group that had been inoculated with pMOK received a second dose of pMOK, the group that had received MIDGE-NLS received VV-Luc in the second booster response dose and the group that received pMOK-LACK received MVA-LACK. Three weeks after the administration of the booster dose the animals were challenged with 5 x 10$^4$ promastygotes of *L. Major* subcutaneously in the paw pad. Lesions at the site of the inoculation and the parasite load in the ganglia that drain the paw pad (popliteal ganglia) measured 7 weeks after the challenge, were considered as protection parameters.

The results obtained by representing the evolution of the size of the lesion with time are shown in Part A of Figure 12. Those corresponding to the parasite load detected in the popliteal ganglia of each one of the groups, expressed as the number of parasites detected per milligram, are shown in Part B of this Figure.

As can be seen in these Figures, the group immunised with pMOK-LACK/MVA-LACK were protected against leishmaniasis produced by *L. Major,* since the mice that were immunised with it hardly developed a lesion. This measurement is correlated with a decrease in the parasite load: they had 4 times less parasites than the control groups.

6.3. <u>Determination of the optimal viral dose of MVA-LACK in heterologous immunisation protocols</u>

After having demonstrated that immunisation with MVA-LACK is a powerful inducer of a cellular immune response and that it is correlated with protection against infection by *Leishmania major,* an additional experiment was carried out to determine the optimal viral dose of MVA-LACK in a heterologous immunisation protocol: a first immunisation (priming) dose based on DNA and a second reinforcement immunisation dose (booster) with recombinant viruses derived from Vaccinia.

Balb/c mice were again used as an animal model, on being a strain highly susceptible to infection by *Leishmania.* Nine weeks old females were inoculated intradermally with 100 $\mu$g of the DNA plasmid vector that expresses the DNA-LACK protein(pCINeo-DNA-LACK) or control DNA. Fifteen days later they received the second immunisation dose, by the intraperitoneal route, with 1 x $10^7$ pfu/mouse or 5 x $10^7$ pfu/mouse of MVA-LACK, 5 x $10^7$ pfu/mouse of VV-LACK or 5 x $10^7$ pfu/mouse of VV-Luc, the latter being administered to the group which had received Control DNA. A control group was included that received PBS in the first dose as well as in the second immunisation dose, which is called N *(naive,* usual name in molecular biology to designate that which has not had contact with anything). The immunised groups are represented in Table 3.

Table 3.- Immunisation groups for the evaluation of the optimum dose of MVA-LACK

|  | INITIAL DOSE | FINAL DOSE |
|---|---|---|
| GROUP 1 | DNA-LACK 100 $\mu$g | MVA-LACK 1 X $10^7$ pfu |
| GROUP 2 | DNA-LACK 100 $\mu$g | MVA-LACK 5 X $10^7$ pfu |
| GROUP 3 | DNA-LACK 100 $\mu$g | VV-LACK 5 X $10^7$ pfu |
| GROUP 4 | DNA CONTROL 100 $\mu$g | VV-LUC 5 X $10^7$ pfu |
| GROUP N | PBS | PBS |

Three weeks after the last immunisation, the animals were challenged with 5 X $10^4$ promastygotes of *L. major* subcutaneously in the foot pad. To ensure the infectivity of the promastygotes, these were isolated using peanut agglutinin ($\beta$-D-galactose-(1$\rightarrow$3)-*N*-acetyl-D-galactosamine, PNA). PNA specifically agglutinates non-infective promastygotes, while it does not agglutinate the metacyclic promastygotes, which is the infective form of *Leishmania.* These infective promastygotes are harvested in the supernatant after differential centrifugation and were used as an infective dose.

Five days after the challenge, 3 animals from each group were sacrificed to evaluate the early immune response against infection by *L. major* in the immunised animals. An ELISPOT assay was carried out to evaluate the number of IL-2 secretor cells for every $10^6$ splenocytes specific for the LACK protein. The results obtained are shown in Figure 13. In this it can be seen that the only group which had significant quantities of IL-2 was the group immunised with DNA-LACK and 1 X $10^7$ pfu of MVA-LACK.

The development of a lesion at the inoculation site was evaluated as a protection parameter, a graph being obtained on the evolution of the size of the lesion and the weeks passed since the challenge, which is shown in Figure 14. Additionally, photographs were taken of the state of the lesion in the 8th week after the challenge on the mice immunised following each one of the different protocols indicated.

As can be seen in the graph in Figure 13, the groups immunised with 1 X $10^7$ pfu of MVA-LACK in the booster dose did not develop a lesion. Of the mice immunised with 5 X $10^7$ pfu of MVA-LACK, one in 5 mice developed a small lesion at the inoculation site from the 7th week after the challenge. From the fourth week after the challenge the lesion presented was significantly different in the groups immunised with recombinant vectors derived from Vaccinia as compared to the controls. The group immunised with 5 X $10^7$ pfu of VV-LACK, had a lesion to a certain degree, although this was significantly different to the control group from the 8th week after the challenge. The lesion in the control groups continued increasing exponentially while in the group immunised with DNA-LACK/VV-LACK the lesion began to stabilise.

The percentages of reduction in the size of the lesion are shown in Table 4.

**Table 4. Reduction in the size of the lesion eight weeks after the challenge**

| INITIAL DOSE | BOOSTER DOSE | SIZE OF LESION | % REDUCTION |
|---|---|---|---|
| DNA-LACK | MVA-LACK 1 X $10^7$ | $0.10 \pm 0.05$ | **97.4 %** |
| DNA-LACK | MVA-LACK 5 X $10^7$ | $0.36 \pm 0.33$ | **90.3 %** |
| DNA-LACK | VV-LACK 5 X $10^7$ | $2.17 \pm 1.64$ | **41.8 %** |
| DNA CONTROL | VV-Luc 5 X $10^7$ | $3.73 \pm 0.23$ | **0 %** |

The photographs of Figure 15 clearly demonstrate that those mice immunised with MVA-LACK showed a high degree of protection against cutaneous leishmaniasis, greater than those immunised with the VV-LACK vector. The grade of the lesion on the right paw is seen in the figure, marked with a "+" sign over each of the photographs to indicate where the promastygotes were inoculated, while the left paw, marked with a "-" sign, acts as a control, with no promastygotes inoculated.

As is observed in the photographs in Figure 15, the animals immunised with DNA-LACK in the first dose, initial response dose, and 1 x $10^7$ pfu of MVA-LACK in the second response booster dose, did not have lesions 8 weeks after the challenge (panel A). When 5 x $10^7$ pfu of MVA-LACK was used in the booster dose, only one out of five mice started to develop a lesion in the seventh week after the challenge (panel B left). In the group that received VV-LACK in the booster, the level of protection was less and lesions were observed in 75% of the animals, although significantly less than the control groups (panel C). The control groups had a lesion at the inoculation site (panels D and E).

The animals of the control groups were sacrificed for ethical reasons 9 weeks after the challenge. At the same time the animals of the groups immunised with DNA-LACK and VV-LACK were also sacrificed. The animals of the groups that received MVA-LACK remained with lesions for at least 11 weeks, and are under observation to analyse whether the protection is maintained over time.

**Example 7.- Tests of protection against _Leishmania infantum_**

Tests were also carried out to evaluate whether the powerful induction of cellular immune response triggered by MVA-LACK when it is administered in the second booster dose of the immunisation, after the subjects had been immunised with a DNA vector which equally expresses the LACK antigen in the first dose, also correlated with the generation of protection against _Leishmania infantum,_ which mainly causes visceral leishmaniasis, using a new heterologous immunisation protocol based on DNA-LACK/MVA-LACK. Given that previous studies on vaccination against leishmaniasis have demonstrated that the murine model can be used to predict the results that might be obtained in vaccination trials carried out on non-human primate models (40, 41, 42), the intradermal murine model was used to test the potential of heterologous vaccination protocols with DNA vectors and recombinant viruses derived from Vaccinia.

7.1. Evaluation of parasite load

Balb/c mice from 4 to 6 weeks old were inoculated intradermally with 100 $\mu$g of the DNA plasmid vector that expressed the LACK protein, DNA-LACK (pCINeo-DNA-LACK) or Control DNA. Fifteen days later they received the second immunisation dose, by the intraperitoneal route, with 1 x $10^7$ pfu/mouse or 5 x $10^7$ pfu/mouse of MVA-LACK or the recombinant virus derived from the Western Reserve wild strain which equally had the LACK antigen inserted in the haemagglutinin locus (VV-LACK). The group that received Control DNA were administered 5 x $10^7$ pfu/mouse of VV-Luc. A control group was included which received PBS in the first and second immunisation dose.

Three and a half weeks after the administration of the booster dose, the mice were infected intradermally in the ear pavilion using 1 x $10^7$ _L. infantum_ metacyclic promastygotes as has been described previously (43). One month after the infection, the parasite loads were evaluated using the analysis by limiting dilution in the immunised and control mouse groups. The evaluations were carried on the spleen, liver and lymphatic drainage nodule. The results obtained, which are shown in Figure 16, in which the mean values obtained from at least 4 mice per group are displayed, demonstrating that the mice immunised following a vaccination protocol with a first response priming dose and a second booster dose using vectors capable of expressing the LACK antigen were protected significantly against infection. The level of protection in each tissue was comparable between the different groups of mice who had received vectors capable of expressing the antigen and it did not differ statistically between the mice that received VV-LACK or MVA-LACK. However, variations were observed in the levels of protection between the different organs, with higher levels of protection being observed in the lymphatic drainage nodule (part C of Figure 16). The level of protection in this organ varied between a reduction factor of 144 to 244 times in the parasite loads compared with control mice. In the spleen (part A of Figure 15) and in the liver (part B of Figure 16) lower levels of protection were observed, which varied between reduction factors of 6 to 9 times in the parasite loads in the liver and 9 to 30 times in the spleen. A small protector effect is also observed in the spleen in the cases where the mice received the control DNA and the VV-Luc virus, which could be due to the low IFN-γ response observed in these animals. Even so, the differences between the parasite loads observed in the mice immunised with VV-Luc and those who received VV-LACK or MVA-LACK were significant (p<.02-.05), which demonstrates a specific effect of the LACK antigen.

7.2. Cellular response and cytokine production

Given that IFN-γ and the TNF-α/LT ratio appears to be involved in the resistance to the infection in murine visceral leishmaniasis (27, 29, 30, 46), while IL-10 appears to be associated with susceptibility (26, 33), the spleens of immunised and non-immunised mice were removed both before proceeding with the challenge with *L. infantum* and one month after having carried it out. With these, an ELISPOT test was carried out to evaluate the number of IFN-γ secretor cells specific for LACK present in every $10^6$ splenocytes, as well as performing assays of the levels of cytokines produced from the supernatant of cultures of the said splenocytes isolated from mice re-stimulated with LACK protein during their culture. The results obtained with the splenocytes extracted before the challenge are shown in Figure 17. Part A, corresponding to the concentration of IFN-γ, in ng/ml, detected in the supernatants of re-stimulated splenocyte cultures, shows that the mice that received 1 x $10^7$ pfu of VV-LACK or 5 x $10^7$ pfu of MVA-LACK seem to produce somewhat higher levels of IFN-γ (100-113 ng/ml), compared with the mice that received 5 x $10^7$ pfu of VV-LACK or 1 x $10^7$ pfu of MVA-LACK (55-67 ng/ml). The evaluation of IFN-γ producer cells specific for LACK carried out by ELISPOT, which is shown in part B of Figure 17, indicates that the number IFN-γ secretor cells correlates with the levels of IFN-γ detected by ELISA, the frequency of IFN-γ producer cells varying between 380 and 640 cells/$10^6$ splenocytes. Significant levels of TNF-α/LT (58 and 134 pg/ml, respectively) were also observed in the mice that had received VV-LACK, while the TNF-α/LT levels produced in response to the LACK antigen by the mice that had received MVA-LACK were lower (27 pg/ml and 8 pg/ml, respectively). These differences in TNF-α induction can reflect, partly, the different capacity of the WR and MVA to induce inflammatory responses and activation of NF-κB, which results in different cytokine profiles: MVA increases the activation of NF-κB while WR appears to inhibit it. The quantities of IL-10 produced by the splenocytes isolated before the challenge, shown in part D of Figure 17, also varied depending on the immunisation protocol used, varying between 0.1 ng/ml in mice who had received 5 x $10^7$ pfu of VV-LACK in the booster dose and 0.7 ng/ml in those that had received 1 x $10^7$ of VV-LACK or MVA-LACK.
The cytokine responses detected in splenocytes isolated one month after the mice were subjected to a challenge with *L. infantum* showed variations between samples parallel to those found before the challenge, although they were somewhat higher.
Significant levels of IL-10 and TNF-α/LT were also produced. The concentrations of IFN-γ detected by ELISA in the supernatants of splenocytes subjected to re-stimulation, the IFN-γ/IL-10 ratio and the reduction factors of the parasite load in the tissues analysed, all these evaluated one month after the challenge with *L. infantum,* are shown below in Table 5, where it can be seen that both the IFN-γ and the IFN-γ/IL-10 ratio appear to correlate with the protection found.

**TABLE 5.- Cytokine levels and reduction factors of the parasite load detected one month after the challenge with *L. infantum***

| Experimental Group | IFN-γ/IL-10 Ratio | IFN-γ (ng/ml ) | Reduction factor of parasite load Spleen Liver Lymphatic nodule | | |
|---|---|---|---|---|---|
| DNA-LACK + 5 X $10^7$ MVA-LACK | 4633 | 204 | 31 | 9 | 244 |
| DNA-LACK + 1 X $10^7$ VV-LACK | 720 | 153 | 18 | 6.5 | 244 |
| DNA-LACK + 1 X $10^7$ MVA-LACK | 271 | 147 | 12.8 | 6.7 | 203 |
| DNA-LACK + 5 X $10^7$ VV-LACK | 92 | 20 | 9.2 | 6 | 144 |

Additionally, the induction of NO/nitric was examined one month after the challenge, as it has been demonstrated that nitric oxide is critical for the leishmanicide activity of murine macrophages (44, 45, 46). It was observed that, in the mice that had received vectors capable of expressing LACK, significant quantities of this antimicrobial agent were detected, which varied between 6 and 7 $\mu$M, whilst in the control groups the NO/nitric levels were much lower, varying between 0.5 $\mu$M and 1 $\mu$M. Therefore, the vaccinated mice, in keeping with the levels of IFN-$\gamma$ detected, had higher levels of inducing the production of nitric oxide and a higher leishmanicide potential. These results are consistent with the protection found in the mice vaccinated with DNA-LACK and VV-LACK or MVA-LACK.

Therefore, the tests described in this example demonstrate that the administration of recombinant viruses derived from Vaccinia capable of expressing the LACK antigen as part of immunisation protocols where a second booster dose is administered, while a DNA vector, which expresses the same antigen, is highly immunogenic and provides protection against *L. infantum* in mice is administered in the first dose, while the protector effect is not achieved with vaccinations with DNA vectors in the two doses. The vector derived from the highly attenuated MVA strain and that derived from the virulent strain of Vaccinia competent for Western Reserve replication gave rise to comparable levels of protection. However, the fact that the highly attenuated MVA strain guarantees higher safety for its use in human beings makes this vector a good candidate to be used for the protection of human beings against visceral leishmaniasis.

### -Example 8 Growth efficiency of MVA-LACK

An additional assay was performed to determine the growth efficiency of the recombinant virus MVA-LACK. To do this, permissive cells were infected (BHK-21) at a multiplicity of infection of 0.01, either with the recombinant virus MVA-LACK or with the parental virus lacking inserts, MVA-WT. At different times post-infection (1, 24, 48, 72h), the virus present in the growth medium was titrated (extracellular) and the virus associated to cells (intracellular) by immunostaining of the virus.

In Figure 18, the results are plotted on graphs as a function of time. Both in the graph showing the intracellular virus (A) and in the graph of extracellular virus (B), there is no growth inhibition of the recombinant viruses MVA-LACK compared with the parental virus.

### -Example 9.-Duration of the protection induced by the administration of DNA-LACK and MVA-LACK

To demonstrate that the immunization protocol based on the administration of DNA-LACK and MVA-LACK is reproducible and that the protection induced by its administration is long-lasting, an experiment was performed with Balb/c mice, which were administered a first initial dose, to trigger the immune response, that contained the plasmidic vector DNA-LACK (pCI-neo_LACK, 100$\mu\mu$g/mouse) and in which the immune response was boosted by administration 14 days later of the MVA-LACK vector ($5^7$ or $5 \times 10^7$ ufp/mouse) or the VV-LACK vector ($5 \times 10^7$ ufp/mouse) Two negative controls were included in the assay: in one of these DNA-LACK was substituted by an "empty" plasmid, lacking the insert with LACK-encoding sequences (DNA-Control), and the recombinant viruses with LACK sequences were substituted by the virus VV-LUC, a virus derived from Vaccinia that contains the luciferase gene inserted at the hemaglutinin site but that lacks the insert with LACK encoding sequences; in the second negative control, the animals were not stimulated with any vector ("naive"), but received PBS in the two immunization doses.

A total of 5 groups were formed of 5 mice each. The different immunization groups and the treatments received are summarized in the following Table 6:

Table 6: Inmunization groups of protection duration test

|  | INITIAL DOSE | BOOSTER DOSE |
|---|---|---|
| **GROUP 1** | DNA-LACK 100 $\mu$g | MVA-LACK $1 \times 10^7$ ufp |
| **GROUP 2** | DNA-LACK 100 $\mu$g | MVA-LACK $5 \times 10^7$ ufp |
| **GROUP 3** | DNA-LACK. 100 $\mu$g | VV-LACK $5 \times 10^7$ ufp |
| **GROUP 4** | DNA-CONTROL 100 $\mu$g | VV-LUC $5 \times 10^7$ ufp |
| **GROUP 5** | PBS | PBS |

Three weeks after administration of the booster dose the immune response was challenged by inoculation of $5 \times 10^4$ metacyclic promastygotes of *Leishmania major,* isolated from stationary cultures of *Leishmania* after incubation with peanut aglutinine. The promastygotes were inoculated subcutaneously in the plantar pad of the right hind leg. The evolution of the lesions in the plantar pad where the inoculate had been introduced was followed, as a parameter to evaluate protection, taking measurements weekly. In contrast to assays described previously in this report, the assay was prolonged for 30 weeks after the challenge, to establish whether the protection conferred by immunisation was extended in time.

The upper part (A) of Figure 19 depicts a graph which represents the evolution of the size of the lesions with time after the challenge. Corroborating the results obtained in the analogous experiments performed, all the animals in the control groups, Group 4 (DNA-control/VV-LUC) and Group 5 (PBS/PBS)developed severe lesions, as can be observed in the photographs shown in part B of Figure 19, taken 8 weeks after the challenge. In week 9, the control animals had to be sacrificed for ethical reasons. The animals that received vectors containing LACK inserts, mice immunized with MVA-LACK, showed, once again, a higher degree of protection than mice immunized with VV-LACK (VV-LACK-HA-in Figure 19). None of the animals immunized with DNA-LACK followed by MVA-LACK had developed lesions 8 weeks after the challenge and only one mouse from the group immunized with MVA-LACK at a dose of $1 \times 10^7$ ufp/mouse started to develop a lesion (3mm) 15 weeks after the challenge.

## -Example 10-Increased protection using adjuvants

To study whether the protection conferred by the vector of the invention could be improved by the addition of adjuvants during the immunization protocol, an experiment was carried out in which mice were immunized with a first dose of DNA-LACK, followed by a dose of MVA-LACK, inoculating animals one day after each of the doses of vaccination vector, with different amounts of adjuvant, namely the protein CD40L(Mega CD40L;m-ACRP30m-CD40L, from APOXIS).

The animals, Balb/c mice, were immunised intradermically on day 0 with 100 μg of plasmid DNA-LACK or of the plasmid DNA-Control, without the LACK insert, and one day later the animals received the adjuvant by the same route (1, 10 or 20μg of Mega CD40L, according to the group). Fifteen days later, the mice received the booster dose with $1 \times 10^7$ ufp/mouse of MVA-LACK or MVA-WT intraperitoneally, and one day later they were inoculated by the same route with different amounts of adjuvant (1, 10 or 20μg of Mega CD40L, depending on the group). Two more control groups were included: to one group, only PBS was added and the other was administered PBS together with the adjuvant, to rule out any possible effect of the adjuvant itself. The different immunisation groups, each composed of 4 mice per group, and the treatments received are summarized in Table 7.

**Tabla 7**: Immunization groups of test with adjuvant

|  | INITIAL DOSE (Week 0) | BOOSTER DOSE (Week 2) | 2nd BOOSTER DOSE (Week 15) |
|---|---|---|---|
| **GROUP 1** | DNA-LACK (100 μg) | MVA-LACK($1\times10^7$ ufp) | MVA-LACK($5\times10^7$ ufp) |
| **GROUP 2** | DNA-LACK (100 μg) Mega CD40L (1 μg) | MVA-LACK ($1 \times 10^7$ ufp) Mega CD40L (1 μg) | - |
| **GROUP 3** | DNA-LACK (100 μg Mega CD40L (10 μg) | MVA-LACK ($1 \times 10^7$ufp) Mega CD40L (10 μg) | MVA-LACK ($5 \times 10^7$ ufp) |
| **GROUP 4** | DNA-LACK (100 μg) Mega CD40L (20 μg) | MVA-LACK ($1 \times 10^7$ ufp) Mega CD40L (20 μg) | MVA-LACK ($5 \times 10^7$ ufp) Mega CD40L (20 μg) |
| **GROUP 5** | DNA-CONTROL (100 μg) Mega CD40L (10 μg) | MVA-WT ($1 \times 10^7$ ufp) Mega CD40L (10 μg) | - |
| **GROUP 6** | PBS | PBS | - |
| **GROUP 7** | PBS Mega CD40L (10 μg) | PBS Mega CD40L (10 μg) | - |

Three weeks after receiving the first booster dose, mice were inoculated with $5 \times 10^4$ metacyclic promastigotes of *L. major.* As shown in Table 7, ten weeks after the challenge (15 weeks after having been administered the initial dose), the mice from groups 1, 3 and 4 received a second booster dose with $5 \times 10^7$ ufp of MVA-LACK; in the case of group 4, together with this second booster dose, the mice also received 20μg of adjuvant, also intraperitoneally. The upper part A of Figure 20 shows a schematic representation of the immunization protocol followed.

The size of the lesions was monitored weekly, obtaining the results shown in the graph appearing in the middle of Figure 20, labelled "B". The graph of the lower part of Figure 20, labelled "C" shows the size of the lesions measured in each of the mice surviving from groups 1,2,3,4,5 and 7, 27 weeks after the challenge. Mice from group 6 that had only received PBS before the challenge, had to be sacrificed 12 weeks after the challenge, as also occurred with one mouse from group 2 after the same period of time, and also with one mouse from group 5 a week later.

Both graphs show that lesions had developed less in mice immunized with DNA-LACK and MVA-LACK than in the control groups, except for the case of the group immunized with 1μg of Mega-CDL40 together with the vaccination vectors DNA-LACK and MVA-LACK (group 2), the group in which all the mice developed lesions with a similar size to those observed in the control groups. The addition of 10μg of Mega-CD40L after immunizations with the vectors DNA-LACK and MVA-LACK (group 3) do not seem to have any effect on the protection, since after giving similar results to those obtained in group 1: only one of the four mice from group 3 developed a lesion, the size of which was approximately 6.5 mm 27 weeks after the challenge. The addition of 20μg of Mega-CD40L after immunisations with the vectors DNA-LACK and MVA-LACK (group 4) did, however, seem to improve the results obtained after immunizing with DNA-LACK and MVA-LACK without adjuvant, since in group 4 only one mouse developed a small lesion, approximately 2 mm in size, 27 weeks after the challenge. However, in the control groups (groups 5 and 7), the mean size of the lesions observed 27 weeks after the challenge was approximately 3mm, although one of the mice had developed a smaller lesion, of around 1 mm.

For the groups immunized with DNA-LACK and MVA-LACK without adjuvant (groups 1 and 3), only one mouse from each group developed a lesion; in group 3, the mouse had to be sacrificed before the 27 week period after challenge had elapsed because of the large size of the lesion. The mice from these two groups that did not develop a lesion (75%), parasitic growth was controlled, as shown by the fact that they did not develop lesions 27 weeks after the challenge and also because they did not present any inflammation of the draining lymphatic nodule. Group 1, to which adjuvant was not administered, administration of the second booster dose after the challenge helped to control parasite replication.

The data obtained seem to indicate both that the administration of a second booster dose is positive in that it increases the immunity produced by previous doses, and also that the administration of vaccination vectors that express the LACK protein is compatible with the administration of adjuvants, although it is preferable to do this.

**Example 11:Dog Vaccination assay**

In order to test the efficacy of the vectors of the invention as vaccines, by administering them to dogs, which are a natural reservoir of the disease, an assay was performed to test for protection against *Leishamania infantum* conferred by vaccination of the vector of the invention MVA-LACK, together with an initial dose of the DNA-LACK plasmid, comparing its efficacy with that of the recombinant virus derived from rVV-LACK vaccinia which includes as an insert the LACK sequence at the thimidine kinase locus, TK (in other words, this corresponds to the vector previously referred to as VV-LACK-TK-in this report).

**11.1 Animals**

Four groups of dogs were used for the experiments described in this assay. A total of 16 animals were chosen from a colony of Beagle dogs, obtained from the Veterinary Science Faculty of Zaragoza University and kept there in conditions designed to exclude any possible contamination with *Leishmania* infection. The dogs were aged between 18 months and 4.5 years old, were well nourished, and had been kept under constant surveillance by veterinary surgeons to control for the appearance of health problems and had received all the routine vaccinations against leptospirosis, distemper, adenovirus-2, hepatitis, parainfluenza and parvovirus (Hipradog 7, Hipra, Amer, Spain) The dogs were also treated with antihelminthic drugs (Dontal Puls, Bayer, Germany). All dogs gave negative results for antibodies against *Leishmania* in tests performed by indirect immunofluorescence (IIF), Direct agglutination assays (DAT) and ELISA.

The dogs were divided into four vaccination groups:

- The first group, used as a negative control, were neither vaccinated nor inoculated with L. *infantum.* Dogs from this group received 0.05 ml of saline solution intravenously in one day (47,48).
- The second group was used as a positive control. The dogs were inoculated with *L. infantum* but were not vaccinated.
- The third group was vaccinated with two doses of the LACK encoding gene, once on day 30 with the DNA-LACK plasmid (100μg) and the second time on day 15 with the recombinant virus derived from vaccinia rVV-LACK ($10^8$ufp/dog).
- The fourth group was inoculated with a dose of DNA-LACK (100μg)on day 30 and a second dose of the LACK encoding gene by the MVA-LACK virus ($10^8$ufp/dog).

Dogs of all the groups, except Group 1 (negative control), were inoculated intravenously, with $10^8$ promastygotes of *Leishmania infantum* in 0.5 ml saline solution, obtained as specified in section 11.2. The day of the inoculation of the parasites was considered as day 0. Table 8 gives a summary of the inoculations received by each group.

**Table 8: Groups for dog vaccination test**

|  | INITIAL DOSE (Day 30) | BOOSTER DOSE (Day 15) | CHALLENGE (Day 0) |
|---|---|---|---|
| **GROUP 1 (5 dogs)** | - | - | 0.5 ml Saline solution |
| **GROUP 2 (3 dogs)** | - | - | *L. infantum* ($1 \times 10^8$ ufp) |
| **GROUP 3 (4 dogs)** | DNA-LACK (100 $\mu$g) | rVV-LACK($1 \times 10^8$ ufp) | *L. infantum* ($1 \times 10^8$ ufp) |
| **GROUP 4 (4 dogs)** | DNA-LACK (100 $\mu$g) | MVA-LACK ($1 \times 10^8$ ufp) | *L. infantum* ($1 \times 10^8$ ufp) |

**11.2** Isolation of parasites for experimental infection, direct agglutination assays and ELISA

The promastygotes of *L.infantum* used to trigger the experimental infection were obtained from a dog in Zaragoza, infected naturally with *L.infantum*, which had not received any treatment (MON 1/MCAN/ES/01/LLM 996, Parasitology Reference Service, Majadahonda, Spain). The parasites were obtained by aspiration of bone marrow and popliteal lymph nodes, they were grown in NNN medium (Novy-Nicolle-McNeal), a medium prepared in two steps, after which they were multiplied in RPMI (Sigma, United Kingdom) supplemented with 2 mM glutamine, 100$\mu$g/ml of streptomycin and 100 U/ml of penicillin containing 10% heat-inactivated fetal calf serum (FCS)(Sigma-United Kingdom). The stationary promastygotes were recovered by centrifugation, resuspended in PBS, adjusted to a concentration of $1 \times 10^8$ufp/0.5 ml) and injected intravenously into the dogs (49,50).

*L.infantum* promastygotes were also grown in RPMI medium containing 10% fetal calf serum at 26°C, to be used in the direct agglutination and in the immunofluorescence assay. The promastygotes were collected at 3600 x g for 10 minutes at 4°C. After rinsing 5 times, 20 volumes of trypsin were added to the sediment (0.4% p/v, from Difco), the mixture was incubated at 37°C for 45 minutes and then rinsed 5 more times in cold Locke solution (NaCl 154 mM, KCl 6 mM, NaHCO$_3$ 2 mM pH 7.7). The cells were counted and resuspended to reach a final concentration of $1 \times 10^8$cells/ml.

The soluble *Leishmania* antigen was prepared by growing the promastygotes in RPMI 1640 medium (Gibco) supplemented with 10% heat-inactivated FCS, 100$\mu$g/ml of streptomycin and 100 UI/ml of penicillin (Gibco). The parasites were collected at the end of the logarithmic phase, rinsed in PBS and fragmented by three freezing-thawing cycles. Then, the parasites were sonicated and centrifuged at 16000 x g for 3 minutes at 4°C, collecting the supernatant. Then, the concentration of proteins was determined using the Bradford method with the Bio-Rad Protein Assay kit (BioRad Laboratories).

11.3 Evaluation of the susceptibility or resistance to infection.

The dogs were checked regularly for signs of parasitic infection and development of the disease by carrying out routine examinations to search for classical clinical signs such as cutaneous lesions, alopecia, the presence of popliteal lymphodenopathies, weight loss, ulceration of the skin and pallor of the mucosa. For the first six months of the experiments, blood samples were taken from the animals every two weeks for biochemical and serology tests and for cytokine mRNA tests. After this, and until the end of the experiment, blood samples were taken monthly. The presence of parasites and possible development of the disease was detected by a direct agglutination test (DAT), as described in section 11.4 (51,52,53), and an indirect immunofluorescence serology test (IIF) with specific anti-*Leishmania* antibodies, as described in the same section.

The parasite loads in the liver and the spleen were quantified by the method of Baumann (54). Briefly, each group of tissue samples was weighed before counting the number of parasites corresponding to 500 cell nuclei in slices of this tissue under the microscope. The total parasite load per organ was determined by the formula

$$\text{Total parasite load} = \text{n}^{\circ} \text{ amastygotes/nucleus} \times \text{weight or organ (mg)} \times 2 \times 10^5$$

and is the average of three independent determinations. The presence or absence of the parasite confirms whether or not there has been an infection.

In addition to the biopsy examinations, an attempt was also made to determine the presence of parasites by the "isolation" technique, which consisted in growing lymphatic tissue of the lymph node in NNN growth medium, to try to grow and isolate the parasite. However, it is difficult to carry out this technique successfully, which is why it was not possible to isolate the parasite in all the cases in which this was detected by biopsy. For this reason, the presence of the parasites obtained by biopsy was considered to be sufficient to make a diagnosis.

11.4 Serological tests of parasitic presence: DAT and IIF

For the DAT test, an equal volume of formaldehyde solution was added to a suspension of *L.infantum* promastygotes ($1 \times 10^8$ cells/ml, prepared for the agglutination test as described in section 11.2) and the mixture was incubated all night at room temperature (RT) The suspension was centrifuged (3600 x g, 10 minutes) to remove the formaldehyde and resuspended in a solution with sodium citrate (NaCl 150 mM, sodium citrate 50 mM, pH 7.4) containing 0.1% (p/v) of Coomassie blue. After incubation for 90 minutes at RT, the excess colouring was removed by centrifugation in the same citrate solution (3600 x g, 10 minutes) and the solution was finally resuspended with citrate containing 0.4% formaldehyde. Samples were kept in the dark at 4°C, ready for use.

The test was performed on a V-shaped 96-well microtitre plate (Costar, US). A total of 50 μl of parasite suspension were added to each well, which had been previously loaded with serial dilutions of dog serum, with an initial dilution of 1:100. The plates were shaken gently for 1 minute and were then incubated at RT in a moist chamber overnight. The presence of blue aggregates was detected by direct observation (two independent measurements), correlated with serum dilutions. Values higher than 1:800 were considered as positive.

For the immunoflourescence assay, the dog serum to be tested is made to react with a preparation of *Leishmania* on a slide, incubated at 4°C for 30-45 minutes and then the preparation is incubated with an anti-dog serum conjugated with fluoresceine, for at least an hour, so that if there is a specific antibody present in the dog serum that can recognise the *Leishmania* parasite, this will be labelled green. Sera with a value of 1/80 or above will be given a positive value (55,56).

The results obtained with each of the dogs can be summarised in Table 9, together with those corresponding to the presence of parasites and development of the infection or not. In this table, cells corresponding to serological tests and tests of parasite presence in which data appear, correspond to dogs for which a positive result has been obtained. The numbers given in brackets correspond to the day on which the corresponding data were stabilised.

**Table 9: Results of parasite presence and disease course in dogs in relation to immunization group**

| GROUP | Dog | Serology | | | Presence of parasites | | | Infection/ Disease |
|---|---|---|---|---|---|---|---|---|
| | | DAT | IIF | | Isolation | Biopsy* | | |
| Positive Control (2) | I8 | 1/6400 (406) | 1/1280(406) | | (426) | (406) | | Yes |
| | D9 | 1/1600 (320) | 1/5120 (320) | | (436) | (330) | | Yes |
| | O1 | 1/800(260) | 1/5120 (260) | | (436) | (290) | | Yes |
| | O2 | 1/6400 (289) | 1/640 (320) | | (426) | (426) | | Yes |
| | O3 | 1/3200 (320) | 1/1240(320) | | (436) | (426) | | Yes |
| Negative Control (1) | O4 | - | - | | - | - | | No |
| | O5 | - | - | | - | - | | No |
| | O6 | - | - | | - | - | | No |
| DNA-LACK/ rVV-LACK (3) | I9 | 1/1600 (410) | 1/160 (350) | | (-) | (436) | | Yes |
| | D4 | - | - | | - | - | | No |
| | D5 | - | - | | - | - | | No |
| | D7 | - | - | | - | - | | No |
| DNA-LACK/ MVA-LACK (4) | I2 | - | - | | - | - | | No |
| | I3 | - | - | | - | - | | No |
| | I7 | - | - | | - | - | | No |
| | D3 | 1/1600 (320) | 1/640 (320) | | - | (436) | | Yes |

### 11.5 Determination of IgG1 and IgG2

Additionally, a test was performed to determine IgG1 and IgG2 in the plasma samples obtained from the blood samples extracted over the study period. Specifically, determination of specific antibodies was carried out by ELISA of the specific antibodies against the soluble *Leishmania* antigen (SLA), obtained as described in section 11.2. Briefly, the ELISA plates were coated with 10 $\mu$g/ml of SLA blocked with PBS containing 1% BSA and afterwards incubated with 100$\mu$l of dog serum diluted 1:100. After rinsing three times, 100$\mu$l/well of the following HRP-conjugated antibodies were added:goat anti-dog IgG1 antibody (1:15000) or a sheep anti-dog IgG1 antibody (1:20000) both from Bethyl Laboratories (Montgomery, TX, US). The antibodies were incubated with the OPD substrate (Zymed laboratories, Invitrogen Immunidetection). Absorbance was read at 450 nm, arbitrarily assigning a value of 1 in each group to the mean value of the samples obtained on day 30 of the experiment and calculating the value n for the remaining samples, corresponding to an increase per one in absorbance compared to the value obtained on day 30.

The results obtained are represented in Figure 21. From this figure it can be observed that the production of IgG1 and IgG2 is similar in both groups of vaccinated dogs, although slightly higher in the dogs vaccinated with the vector from the Western Reserve Vaccinia strain (rVV-LACK). Activation of the Th1/Th2 system is similar in both cases. In the case of IgG1, after six months the values are approaching control values, whereas the levels of IgG2, associated with the protective response, remain high and above control values (positive and negative) until the end of the experiment, both in dogs immunized with the rVV-LACK vector and in those immunized with MVA-LACK. This suggests that vaccination with DNA-LACK/MVA-LACK induces a Th1 protective response against the infection as occurs with vaccination with DNA-LACK/rVV-LACK.

Taken together, the results indicate that vaccination with DNA-LACK and MVA-LACK confer, to the dogs they are administered to, protection against the development of the disease similar to that conferred by vaccination with DNA-LACK and rVV-LACK, obtaining in both cases experimental protection of at least 75% compared to positive controls, of which 100% presented the infection and clear clinical signs of the disease. The experiment confirms the validity of MVA-LACK as an alternative to the recombinant vectors derived from virulent strains of Vaccinia (as is the case of rVV-LACK) for the vaccination of mammals suspectible to being infected with Leishmania, especially for dogs. Moreover, since MVA-LACK is an attenuated virus it represents a much safer alternative to these other vectors.

## REFERENCES

1. Ahmed, S. B., C. Bahloul, C. Robbana, S. Askri, and K. Dellagi. 2004. A comparative evaluation of different DNA vaccine candidates against experimental murine leishmaniasis due to L. major. Vaccine 22:1631-9.

2. Altenburger, W., C. P. Suter, and J. Altenburger. 1989. Partial deletion of the human host range gene in the attenuated vaccinia virus MVA. Arch Virol 105:15-27.

3. Berrahal, F., C. Mary, M. Roze, A. Berenger, K. Escoffier, D. Lamouroux, and S. Dunan. 1996. Canine leishmaniasis: identification of asymptomatic carriers by polymerase chain reaction and immunoblotting. Am J Trop Med Hyg 55:273-7.

4. Blanchard, T. J., A. Alcami, P. Andrea, and G. L. Smith. 1998. Modified vaccinia virus Ankara undergoes limited replication in human cells and lacks several immunomodulatory proteins: implications for use as a human vaccine. J Gen Virol 79 (Pt 5): 1159-67.

5. Caver, T. E., T. D. Lockey, R. V. Srinivas, R. G. Webster, and J. L. Hurvitz. 1999. A novel vaccine regimen utilizing DNA, vaccinia virus and protein immunizations for HIV-1 envelope presentation. Vaccine 17:1567-72.

6. Drexler, I., K. Heller, B. Wahren, V. Erfle, and G. Sutter. 1998. Highly attenuated modified vaccinia virus Ankara replicates in baby hamster kidney cells, a potential host for virus propagation, but not in various human transformed and primary cells. J Gen Virol 79 (Pt 2):347-52.

7. Gonzalez-Aseguinolaza, G., S. Taladriz, A. Marquet, and V. Larraga. 1999. Molecular cloning, cell localization and binding affinity to DNA replication proteins of the p36/LACK protective antigen from Leishmania infantum. Eur J Biochem 259:909-16.

8. Gonzalo, R. M., G. del Real, J. R. Rodriguez, D. Rodriguez, R. Heljasvaara, P. Lucas, V. Larraga, and M. Esteban. 2002. A heterologous prime-boost regime using DNA and recombinant vaccinia virus expressing the Leishmania infantum P36/LACK antigen protects BALB/c mice from cutaneous leishmaniasis. Vaccine 20:1226-31.

9. Gonzalo, R. M., J. R. Rodriguez, D. Rodriguez, G. Gonzalez-Aseguinolaza, V. Larraga, and M. Esteban. 2001. Protective immune response against cutaneous leishmaniasis by prime/booster immunization regimens with vaccinia virus recombinants expressing Leishmania infantum p36/LACK and IL-12 in combination with purified p36. Microbes Infect 3:701 - 11.

10. Gradoni, L. 2001. An update on antileishmanial vaccine candidates and prospects for a canine Leishmania vaccine. Vet Parasitol 100:87-103.

11. Gurunathan, S., D. L. Sacks, D. R. Brown, S. L. Reiner, H. Charest, N. Glaichenhaus, and R. A. Seder. 1997. Vaccination with DNA encoding the immunodominant LACK parasite antigen confers protective immunity to mice infected with Leishmania major. J Exp Med 186:1137-47.

12. Hanke, T., T. J. Blanchard, J. Schneider, C. M. Hannan, M. Becker, S. C. Gilbert, A. V. Hill, G. L. Smith, and A. McMichael. 1998. Enhancement of MHC class I-restricted peptide-specific T cell induction by a DNA prime/MVA boost vaccination regime. Vaccine 16:439-45.

13. Hanke, T., and A. McMichael. 1999. Pre-clinical development of a multi-CTL epitope-based DNA prime MVA boost vaccine for AIDS. Immunol Lett 66:177-81.

14. Hanke, T., V. C. Neumann, T. J. Blanchard, P. Sweeney, A. V. Hill, G. L. Smith, and A. McMichael. 1999. Effective induction of HIV-specific CTL by multi-epitope using gene gun in a combined vaccination regime. Vaccine 17:589-96.

15. Kent, S. J., A. Zhao, S. J. Best, J. D. Chandler, D. B. Boyle, and I. A. Ramshaw. 1998. Enhanced T-cell immunogenicity and protective efficacy of a human immunodeficiency virus type 1 vaccine regimen consisting of consecutive priming with DNA and boosting with recombinant fowlpox virus. J Virol 72: 10180-8.

16. Kim, J. J., M. L. Bagarazzi, N. Trivedi, Y. Hu, K. Kazahaya, D. M. Wilson, R. Ciccarelli, M. A. Chattergoon, K. Dang, S. Mahalingam, A. A. Chalian, M. G. Agadjanyan, J. D. Boyer, B. Wang, and D. B. Weiner. 1997. Engineering of in vivo immune responses to DNA immunization via codelivery of costimulatory molecule genes. Nat Biotechnol 15:641-6.

17. Lanotte, G., J. A. Rioux, J. Perieres, and Y. Vollhardt. 1979. [Ecology of leishmaniasis in the south of France. 10. Developmental stages and clinical characterization of canine leishmaniasis in relation to epidemiology. (author's transl)]. Ann Parasitol Hum Comp 54:277-95.

18. Li, S., M. Rodrigues, D. Rodriguez, J. R. Rodriguez, M. Esteban, P. Palese, R. S. Nussenzweig, and F. Zavala. 1993. Priming with recombinant influenza virus followed by administration of recombinant vaccinia virus induces CD8+ T-cell-mediated protective immunity against malaria. Proc Natl Acad Sci U S A 90:5214-8.

19. Liew, F. Y., and C. A. O'Donnell. 1993. Immunology of leishmaniasis. Adv Parasitol 32:161-259.

20. Lopez-Fuertes, L., E. Perez-Jimenez, A. J. Vila-Coro, F. Sack, S. Moreno, S. A. Konig, C. Junghans, B. Wittig, M. Timon, and M. Esteban. 2002. DNA vaccination with linear minimalistic (MIDGE) vectors confers protection against Leishmania major infection in mice. Vaccine 21:247-57.

21. Lopez-Velez, R., J. A. Perez-Molina, A. Guerrero, F. Baquero, J. Villarrubia, L. Escribano, C. Bellas, F. Perez-Corral, and J. Alvar. 1998. Clinicoepidemiologic characteristics, prognostic factors, and survival analysis of patients coinfected with human immunodeficiency virus and Leishmania in an area of Madrid, Spain. Am J Trop Med Hyg 58:436-43.

22. Mauricio, I. L., J.R Stothard, and M. A. Miles. 2000. The strange case of Leishmania chagasi. Parasitol Today 16:188-9.

23. Mayr, A., H. Stickl, H. K. Muller, K. Danner, and H. Singer. 1978. [The smallpox vaccination strain MVA: marker, genetic structure, experience gained with the parenteral vaccination and behavior in organisms with a debilitated defence mechanism (author's transl)]. Zentralbl Bakteriol [B] 167:375-90.

24. McMahon-Pratt, D., D. Rodriguez, J. R. Rodriguez, Y. Zhang, K. Manson, C. Bergman, L. Rivas, J. F. Rodriguez, K. L. Lohman, N. H. Ruddle, et al. 1993. Recombinant vaccinia viruses expressing GP46/M-2 protect against Leishmania infection. Infect Immun 61:3351-9.

25. Meyer, H., G. Sutter, and A. Mayr. 1991. Mapping of deletions in the genome of the highly attenuated vaccinia virus MVA and their influence on virulence. J Gen Virol 72 (Pt 5):1031-8.

26. Paoletti, E. 1996. Applications of pox virus vectors to vaccination: an update. Proc Natl Acad Sci U S A 93: 11349-53.

27. Ramirez, J. C., M. M. Gherardi, and M. Esteban. 2000. Biology of attenuated modified vaccinia virus Ankara recombinant vector in mice: virus fate and activation of B- and T-cell immune responses in comparison with the Western Reserve strain and advantages as a vaccine. J Virol 74:923-33.

28. Robinson, H. L., D. C. Montefiori, R. P. Johnson, K. H. Manson, M. L. Kalish, J. D. Lifson, T. A. Rizvi, S. Lu, S. L. Hu, G. P. Mazzara, D. L. Panicali, J. G. Hemdon, R. Glickman, M. A. Candido, S. L. Lydy, M. S. Wyand, and H. M. McClure. 1999. Neutralizing antibody-independent containment of immunodeficiency virus challenges by DNA priming and recombinant pox virus booster immunizations. Nat Med 5:526-34.

29. Sacks, D., and N. Noben-Trauth. 2002. The immunology of susceptibility and resistance to Leishmania major in mice. Nat Rev Immunol 2:845-58.

30. Sedegah, M., T. R. Jones, M. Kaur, R. Hedstrom, P. Hobart, J. A. Tine, and S. L. Hoffman. 1998. Boosting with recombinant vaccinia increases immunogenicity and protective efficacy of malaria DNA vaccine. Proc Natl Acad Sci U S A 95:7648-53.

31. Solano-Gallego, L., J. Llull, G. Ramos, C. Riera, M. Arboix, J. Alberola, and L. Ferrer. 2000. The Ibizian hound presents a predominantly cellular immune response against natural Leishmania infection. Vet Parasitol 90:37-45.

32. Sutter, G., and B. Moss. 1992. Nonreplicating vaccinia vector efficiently expresses recombinant genes. Proc Natl Acad Sci U S A 89:10847-51.

33. Tapia, E., E. Perez-Jimenez, L. Lopez-Fuertes, R. Gonzalo, M. M. Gherardi, and M. Esteban. 2003. The combination of DNA vectors expressing IL- 12 + IL- 18 elicits high protective immune response against cutaneous leishmaniasis after priming with DNA-p36/LACK and the cytokines, followed by a booster with a vaccinia virus recombinant expressing p36/LACK. Microbes Infect 5:73-84.

34. Xu, D., and F. Y. Liew. 1994. Genetic vaccination against leishmaniasis. Vaccine 12:1534-6.

35. Zavala, F., M. Rodrigues, D. Rodriguez, J. R. Rodriguez, R. S. Nussenzweig, and M. Esteban. 2001. A striking property of recombinant poxviruses: efficient inducers of in vivo expansion of primed CD8(+) T cells. Virology 280: 155-9.

36. Melby, P.C., J. Yang, W. Zhao, L.E. Pérez and J. Cheng. 2001. Leishmania donovani p36(LACK) DNA vaccine is highly immunogenic but not protective against experimental visceral leishmaniasis. Infect Immun 69:4719-4725.

37. Moss, B. 1996. Genetically engineered poxviruses for recombinant gene expression, vaccination, and safety. Proc. Natl. Acad. Sci USA 93:11341-11348.

38. Seki, M., M. Oie, Y. Ichihashi, and H. Shida. 1990. Hemadsoption and fusion inhibition activities of hemagglutinin analyzed by vaccinia virus mutants. Virology 175, 372-384.

39. Chakrabarti, S. J.R. Sisler, B. Moss. 1997. Compact, synthetic, vaccina virus early/late promoter for protein expression. BioTechniques 23:1094-1097.

40. Belli, S., A. Formenton, T. Noll, A. Ivens, R. Jacquet, C. Desponds, D. Hofer, and N. Fasel. 1999. Leishmania major. histone H1 gene expression from the sw3 locus. Exp. Parasitol 91:151-160.

41. Campos-Neto, A., R. Porozzi, K. Greeson, R.M. Coler, J.R. Webb, Y.A. Seiky, S.G. Reed, and G. Grimaldi, Jr. 2001. Protection against cutaneous leishmaniasis induced by recombinant antigens in murine and nonhuman primate models of the human disease. Infect. Immun. 69:4103-4108.

42. Masina, S., M.G.M., S.O. Demotz, and N.J. Fasel. 2003. Protection against cutaneous leishmaniasis in outbred brevet monkeys using a recombinat histone H1 antigen. J. Infect. Dis. 188:1250-1257.

43. Ahmed, S., M. Colmenares, L. Soong, K. Goldsmith-Pestana, L. Munstermann, R. Molina and D. McMahon-Pratt. 2003. Intradermal infection model for patogénesis and vaccine studies of murine visceral leishmaniasis. Infect Immun 71 :401-10.

44. Bhakuni, V., U.K Singha, G.P. Dutta, H.B. Levy, and R.K. Maheshawari. 1996. Killing of Leishmania donovani amastigotes by poly ICLC in hamsters. J. Interferon Cytokine Res. 16:321-325.

45. Green, S.J., M.S. Meltzer, J.B. Hibbs, Jr., and C.A, Nacy. 1990. Activated macrophages destroy intracellular Leishmania major amastigotes by and L-arginine-dependent killing mechanism. J. Immunol 144:278-283.

46. Liew, F., Y.S. Millot, C. Parkinson, R.M. Palmer, and S. Moncada. 1990. Macropage killing of Leishmania parasite in vivo is mediated by nitric oxide from L-arginine. J. Immunol. 144:4791-4797.

47.Martínez-Moreno, A, Martinez-Cruz MS, Blanco A, Hernández-Rodriguez S. 1993. Inmunological histological study of T-and- B-lymphocyte activity in canine visceral leishmaniasis. Vet. Parasitol 51:49-59.

48.Riera, C, Valladares JE, Gállego M, et al.1999. Serological and parasitological follow-up in dogs experimentally infected with Leishmania infantum and treated with menglubine antimoniate. Vet Parasitol 84:33-47.

49. Evans D. 1989. Handbook on isolation characterization and cryopreservation of Leishmania. En: UNDP/world Bank/WHO special program for research and training in tropical diseases.

50. WHO Manual on visceral leishmaniasis control. WHO/LEISH/96.40. Ginebra (Suiza):1996; p. 51-70.

51. Boelaert M; El Safi S, Mousa H, et al. 1999. Multi-centre evaluation of repeatability and reproducibility of the direct agglutination test for visceral leishmaniasis. Trop Med Int Health 4(1):31-7.

52. Boelaert M; El Safi S, Jacquet D, Van der Stuyf P, Le Ray D, De Muynck D. 1999.Operational validation of the direct agglutination test for diagnosis of visceral leishmaniasis.Am J Trop Med Hyg 4(1 ):129-34.

53. Boelaert M, El Safi S, Goethebeur E, Gomes-Pereira S, Le Ray D, van der Stuyf P. 1999. Latent class analysis permits unbiased estimates of the validity of DAT for the diagnosis of visceral leishmaniasis. Trop Med Int Health4 (5):395-401.

54. Baumann RJ, Hanson WL, McCaan PP, Sjodersma A, Bionti AJ.1990. Suppression of both antimony-susceptible and antimony-resistant Leishmania donovani by a bis(benzyl)polyamine analogue. Antimicrob Agents Chemother 34:722-7

55.Tesouro MA et al 1992. Información Veterinari 127:34-40.

56.Ashford DA et al. 1995. Am. J. Trop. Med. Hyg. 53:251-255

**List of sequences**

**EP 1 916 306 B1**

<110> CONSEJO SUPERIOR DE INVESTIGACIONES CIENTÍFICAS

<120> RECOMBINANT VECTORS BASED ON THE MODIFIED VACCINIA VIRUS ANKARA (MVA) AS VACCINES AGAINST LEISHMANIASIS

<130> PCT-278
<160> 6
<210> 1
<211> 20
<212> DNA
<213> Artificial sequence
<220>
<221> Primer
<223> TK-L

<400> 1
tgattagttt gatgcgattc          20

<210> 2
<211> 17
<212> DNA
<213> Artificial sequence
<220>
<221> Primer
<223> TK-R

<400> 2
tgtccttgat acggcag          17

<210> 3
<211> 18
<212> DNA
<213> Artificial sequence
<220>
<221> Primer
<223> HA-1

<400> 3
gtcacgtgtt accacgca          18

<210> 4
<211> 18
<212> DNA
<213> Artificial sequence
<220>
<221> Primer
<223> HA-2

<400> 4
gatccgcatc atcggtgg          18

<210> 5
<211> 18
<212> DNA
<213> Artificial sequence
<220>
<221> Primer
<223> HA-MVA

<400> 5
tgacacgatt accaatac        18


<210> 6
<211> 939
<212> DNA
<213> Leishmania infantum
<220>
<221> MVA-LACK Insert
<223> LACK


<400> 6


```
atgaactacg agggtcacct gaagggccac cgcggatggg tcacctccct ggcctgcccg 60
cagcaggcgg ggtcgtacat caaggtggtg tcgacgtcgc gcgatggcac ggccatctcg 120
tggaaagcca accccgaccg ccacagcgtg gacagcgact acggtctgcc gagccaccgc 180
ctcgagggcc acaccggctt cgtgtcgtgt gtgtcgctgg cccacgccac cgactacgcg 240
ctgaccgcgt cctgggaccg ctccatccgc atgtgggacc tgcgcaatgg ccagtgccag 300
cgcaagttcc tgaagcacac caaggacgtg ctcgccgtcg ccttctcgcc ggacgaccgc 360
ctgatcgtgt ccgcgggccg cgacaacgtg atccgcgtgt ggaacgtggc gggcgagtgc 420
atgcacgagt tcctgcgcga cggccacgag gactgggtga gcagcatctg tttctcgccg 480
tcgctggagc atccgatcgt ggtgtccggc agctgggaca acaccatcaa ggtatggaac 540
gtgaacgggg gcaagtgtga gcgcacgctc aagggccaca gcaactacgt gtccacggtg 600
acggtgtcgc cagacgggtc gctgtgcgcg tccggcggca aggacggcgc ggcgctgctg 660
```

```
tgggacctga gcaccggcga gcagctgttc aagatcaacg tggagtcgcc catcaaccag 720
atcgccttct cgcccaaccg cttctggatg tgcgtcgcga cggagaggtc tctgtccgtg 780
tacgacctgg agagcaaggc tgtgattgcg gagctgacgc cggacggcgc gaagccgtcc 840
gagtgcatct ccattgcctg gtccgccgac ggcaacactc tgtactccgg tcacaaggac 900
aacctgatcc gcgtgtggtc catctccgac gccgagtaa            939
```


**Claims**

1. A recombinant MVA virus vector comprising a coding sequence of the LACK protein, **characterized in that** the coding sequence of the LACK protein is contained in a DNA fragment which is inserted in the haemagglutinin locus of the MVA genome between the right and left flanking regions of the haemagglutinin (HA) gene, in which DNA fragment there is an early/late pE/L synthetic promoter to which is joined the coding sequence of the LACK protein in a way that the promoter directs the expression of the said coding sequence to give rise to the LACK protein.

2. A recombinant MVA virus vector according to claim 1, in which the coding sequence of the LACK protein is that of the *Leishmania infantum* species.

3. A recombinant MVA virus vector according to claim 2, in which the coding sequence of the LACK protein gives rise to the expression of a form of LACK protein which contains all its amino acids.

4. A recombinant MVA virus vector according to claim 1 comprising the coding sequence of the LACK protein of *Leishmania infantum,* **characterized in that** the coding sequence of the LACK protein is contained in a DNA fragment which is inserted in the haemagglutinin locus of the MVA genome between the right and left flanking regions of the haemagglutinin (HA) gene, in which DNA fragment there are two Vaccinia promoters placed in the opposite direction and located in a part of the DNA fragment further from the ends, which promoters are a Vaccinia p7.5 promoter to which is joined a gene of β-gal in a way that the p7.5 promoter directs its expression, and the early/late pE/L synthetic promoter to which is joined the coding sequence of the LACK protein in a way that the promoter directs the expression of the said coding sequence to give rise to the LACK protein

5. A composition which comprises a recombinant MVA virus vector according to any one of claims 1 to 4 and, optionally, at least one pharmaceutical acceptable adjuvant or vehicle.

6. A composition according to claim 5, in which the protein CD40L is present.

7. Use of a recombinant MVA virus vector according to any one of claims 1 to 4 for the preparation of a drug destined to be administered to a mammal susceptible to being infected by a species of the *Leishmania* genus, for the prevention or treatment in the said mammal of a disease caused by a species of the *Leishmania* genus.

8. Use according to claim 7, in which the disease corresponds to visceral leishmaniasis.

9. Use according to claim 8, in which the visceral leishmaniasis is caused by *Leishmania infantum.*

10. Use according to claim 9, in which the mammal susceptible to being infected by the said species of the *Leishmania* genus for which the drug is destined is a human being.

11. Use according to claim 10, in which the disease corresponds to cutaneous leishmaniasis.

12. Use according to claim 11, in which the cutaneous leishmaniasis is caused by *Leishmania major.*

13. Use according to claim 12, in which the mammal susceptible to being infected by the said species of the *Leishmania* genus for which the drug is destined is a human being.

14. Use according to any one of claims 7 to 13 in which the drug is destined to be administered in a single vaccination dose.

15. Use according to any one of claims 7 to 13 in which the drug is destined to be administered in at least one of the vaccination doses which constitutes a vaccination protocol constituted by at least two vaccination doses, each one of which is administered separated by a time period.

16. Use according to claim 15 in which the drug is destined to be administered in the first vaccination dose which triggers the immune response as well as in at least one of the vaccination doses after the first.

17. Use according to claim 15 in which the drug is destined to be administered in only the first vaccination dose which triggers the immune response, vaccination doses after the first one being absent.

18. Use according to claim 17 in that the drug is destined to be administered in only the first vaccination dose which triggers the immune response, at least one of the vaccination doses subsequent to the first of which the drug is absent containing a different recombinant vector that is also a system of LACK protein expression or an immunogenic fragment of the same.

19. Use according to claim 15 in which the drug is destined to be administered in at least one of the vaccination doses subsequent to the first, being absent from the first vaccination dose.

20. Use according to claim 19 in which the drug is destined to be administered in at least one of the vaccination doses subsequent to the first, the first vaccination dose from which the drug is absent containing a different recombinant vector that is also a system of LACK protein expression or an immunogenic fragment of the same.

21. Use according to claim 20 in which the drug is destined to be administered in at least one of the vaccination doses subsequent to the first, the first vaccination dose from which the drug is absent containing a naked DNA that is also a system of LACK protein expression or an immunogenic fragment of the same.

22. Use according to claim 7, in which the mammal susceptible to infection by a species of the *Leishmania* genus for which the drug is destined is a dog.

23. Use according to claim 22, in which the leishmaniasis is caused by *Leishmania infantum.*

**Patentansprüche**

1. Rekombinanter MVA-Virus-Vektor umfassend eine codierende Sequenz des LACK-Proteins, **dadurch gekennzeichnet, dass** die codierende Sequenz des LACK-Proteins in einem DNA-Fragment enthalten ist, das in den Haemagglutinin-Locus des MVA-Genoms zwischen den rechten und linken flankierenden Regionen des Haemagglutinin (HA)-Gens inseriert ist, wobei in dem DNA-Fragment ein early/late pE/L-synthetischer Promotor ist, mit dem die codierende Sequenz des LACK-Proteins so verbunden ist, dass der Promotor die Expression der codierenden Sequenz steuert, so dass das LACK-Protein gebildet wird.

2. Rekombinanter MVA-Virus-Vektor nach Anspruch 1, wobei die codierende Sequenz des LACK-Proteins die der *Leishmania infantum-Spezies* ist.

3. Rekombinanter MVA-Virus-Vektor nach Anspruch 2, wobei die codierende Sequenz des LACK-Proteins zu der Expression einer Form des LACK-Proteins führt, die alle seine Aminosäuren enthält.

4. Rekombinanter MVA-Virus-Vektor nach Anspruch 1, umfassend die codierende Sequenz des LACK-Proteins von *Leishmania infantum,* **dadurch gekennzeichnet, dass** die codierende Sequenz des LACK-Proteins in einem DNA-Fragment enthalten ist, das in den Haemagglutinin-Locus des MVA-Genoms zwischen den rechten und linken flankierenden Regionen des Haemagglutinin (HA)-Gens inseriert ist, wobei in dem DNA-Fragment zwei Vaccinia-Promotoren in entgegengesetzter Orientierung platziert und in einem Bereich des DNA-Fragments weiter von den Enden lokalisiert sind, wobei die Promotoren ein Vaccinia p7.5-Promotor, der mit einem β-gal-Gen so verbunden ist, dass der p7.5-Promotor dessen Expression steuert, und der early/late pE/Lsynthetische Promotor sind, mit dem die codierende Sequenz des LACK-Proteins so verbunden ist, dass der Promotor die Expression der codierenden Sequenz steuert, so dass das LACK-Protein gebildet wird.

5. Mittel umfassend einen rekombinanten MVA-Virus-Vektor nach einem der Ansprüche 1 bis 4 und, gegebenenfalls, mindestens einen pharmazeutisch verträglichen Hilfsstoff oder Träger.

6. Mittel nach Anspruch 5, in dem das CD40L-Protein vorhanden ist.

7. Verwendung eines rekombinanten MVA-Virus-Vektors nach einem der Ansprüche 1 bis 4 für die Herstellung eines Medikaments, das dazu bestimmt ist, einem Säuger verabreicht zu werden, der empfänglich für eine Infektion mit einer Spezies der *Leishmania*-Gattung ist, zur Vorbeugung oder Behandlung einer Krankheit in dem Säuger, die durch eine Spezies der *Leishmania*-Gattung verursacht wird.

8. Verwendung nach Anspruch 7, wobei die Krankheit viszeraler Leishmaniose entspricht.

9. Verwendung nach Anspruch 8, wobei die viszeraler Leishmaniose durch *Leishmania infantum* verursacht wird.

10. Verwendung nach Anspruch 9, wobei der Säuger, der empfänglich ist für eine Infektion durch die Spezies der *Leishmania*-Gattung, für die das Medikament bestimmt ist, ein Mensch ist.

11. Verwendung nach Anspruch 10, wobei die Krankheit kutaner Leishmaniose entspricht.

12. Verwendung nach Anspruch 11, wobei die kutane Leishmaniose durch *Leishmania major* verursacht wird.

13. Verwendung nach Anspruch 12, wobei der Säuger, der empfänglich ist für eine Infektion durch die Spezies der *Leishmania*-Gattung, für die das Medikament bestimmt ist, ein Mensch ist.

14. Verwendung nach einem der Ansprüche 7 bis 13, wobei das Medikament dazu bestimmt ist, in einer einzelnen Impfdosis verabreicht zu werden.

15. Verwendung nach einem der Ansprüche 7 bis 13, wobei das Medikament dazu bestimmt ist, in mindestens einer der Impfdosen eines Impfprotokolls, das aus mindestens zwei Impfdosen besteht, verabreicht zu werden, wobei jede davon durch ein Zeitintervall getrennt verabreicht wird.

16. Verwendung nach Anspruch 15, wobei das Medikament dazu bestimmt ist, sowohl in der ersten Impfdosis verabreicht zu werden, die die Immunantwort auslöst, als auch in mindestens einer der Impfdosen, die nach der ersten folgen.

**17.** Verwendung nach Anspruch 15, wobei das Medikament dazu bestimmt ist, nur in der ersten Impfdosis verabreicht zu werden, die die Immunantwort auslöst, wobei es nach der ersten Impfdosis keine weiteren Impfdosen gibt.

**18.** Verwendung nach Anspruch 17, wobei das Medikament dazu bestimmt ist, nur in der ersten Impfdosis verabreicht zu werden, die die Immunantwort auslöst, wobei mindestens eine der weiteren Impfdosen nach der ersten, die kein Medikament aufweist, einen anderen rekombinanten Vektor beinhaltet, der auch ein System für LACK-Protein-Expression oder eines immunogenen Fragments desselben ist.

**19.** Verwendung nach Anspruch 15, wobei das Medikament dazu bestimmt ist, in mindestens einer der Impfdosen, die auf die erste folgen, verabreicht zu werden, wobei das Medikament in der ersten Impfdosis fehlt.

**20.** Verwendung nach Anspruch 19, wobei das Medikament dazu bestimmt ist, in mindestens einer der Impfdosen, die auf die erste folgen, verabreicht zu werden, wobei die erste Impfdosis, in der das Medikament fehlt, einen anderen rekombinanten Vektor beinhaltet, der auch ein System für LACK-Protein-Expression oder eines immunogenen Fragment desselben ist.

**21.** Verwendung nach Anspruch 20, wobei das Medikament dazu bestimmt ist, in mindestens einer der Impfdosen, die auf die erste folgen, verabreicht zu werden, wobei die erste Impfdosis, in der das Medikament fehlt, eine nackte DNA beinhaltet, die auch ein System für LACK-Protein-Expression oder eines immunogenen Fragments desselben ist.

**22.** Verwendung nach Anspruch 7, wobei der Säuger, der empfänglich ist für eine Infektion durch eine Spezies der *Leishmania*-Gattung, für die das Medikament bestimmt ist, ein Hund ist.

**23.** Verwendung nach Anspruch 22, wobei die Leishmaniose durch *Leishmania infantum* verursacht ist.


**Revendications**

**1.** Vecteur du virus MVA recombinant comprenant une séquence codante de la protéine LACK, **caractérisé en ce que** la séquence codante de la protéine LACK est contenue dans un fragment d'ADN qui est inséré dans le locus de l'hémagglutinine du génome du MVA entre les régions flanquantes droite et gauche du gène de l'hémagglutinine (HA), dans lequel fragment d'ADN, il y a un promoteur synthétique précoce/tardif pE/L auquel est jointe la séquence codante de la protéine LACK de telle manière que le promoteur dirige l'expression de ladite séquence codante pour donner naissance à la protéine LACK.

**2.** Vecteur du virus MVA recombinant selon la revendication 1, dans lequel la séquence codante de la protéine LACK est celle de l'espèce *Leishmania infantum.*

**3.** Vecteur du virus MVA recombinant selon la revendication 2, dans lequel la séquence codante de la protéine LACK donne naissance à l'expression d'une forme de la protéine LACK qui contient tous ses acides aminés.

**4.** Vecteur du virus MVA recombinant selon la revendication 1, comprenant la séquence codante de la protéine LACK de *Leishmania infantum,* **caractérisé en ce que** la séquence codante de la protéine LACK est contenue dans un fragment d'ADN qui est inséré dans le locus de l'hémagglutinine du génome du MVA entre les régions flanquantes droite et gauche du gène de l'hémagglutinine (HA), dans lequel fragment d'ADN, il y a deux promoteurs de la vaccine placés dans la direction opposée et localisés dans une partie du fragment d'ADN plus loin à partir des extrémités, lesquels promoteurs sont un promoteur p7.5 de la vaccine auquel est joint un gène de la β-gal de telle manière que le promoteur p7.5 dirige son expression, et le promoteur synthétique précoce/tardif pE/L auquel est jointe la séquence codante de la protéine LACK de telle manière que le promoteur dirige l'expression de ladite séquence codante pour donner naissance à la protéine LACK.

**5.** Composition qui comprend un vecteur du virus MVA recombinant selon l'une quelconque des revendications 1 à 4, et, éventuellement, au moins un adjuvant ou solvant acceptable en pharmacie.

**6.** Composition selon la revendication 5, dans laquelle la protéine CD40L est présente.

**7.** Utilisation d'un vecteur du virus MVA recombinant selon l'une quelconque des revendications 1 à 4, pour la préparation d'un médicament destiné à être administré à un mammifère susceptible d'être infecté par une espèce du genre *Leishmania,* pour la prévention ou le traitement chez ledit mammifère d'une maladie provoquée par une espèce du genre *Leishmania.*

8. Utilisation selon la revendication 7, dans laquelle la maladie correspond à une leishmaniose viscérale.

9. Utilisation selon la revendication 8, dans laquelle la leishmaniose viscérale est provoquée par *Leishmania infantum.*

10. Utilisation selon la revendication 9, dans laquelle le mammifère susceptible d'être infecté par ladite espèce du genre *Leishmania* pour laquelle le médicament est destiné, est un être humain.

11. Utilisation selon la revendication 10, dans laquelle la maladie correspond à une leishmaniose cutanée.

12. Utilisation selon la revendication 11, dans laquelle la leishmaniose cutanée est provoquée par *Leishmania major.*

13. Utilisation selon la revendication 12, dans laquelle le mammifère susceptible d'être infecté par ladite espèce du genre *Leishmania* pour laquelle le médicament est destiné, est un être humain.

14. Utilisation selon l'une quelconque des revendications 7 à 13, dans laquelle le médicament est destiné à être administré en une dose de vaccination unique.

15. Utilisation selon l'une quelconque des revendications 7 à 13, dans laquelle le médicament est destiné à être administré dans au moins l'une des doses de vaccination qui constitue un protocole de vaccination constitué par au moins deux doses de vaccination, chaque administration d'une dose étant séparée par une période de temps.

16. Utilisation selon la revendication 15, dans laquelle le médicament est destiné à être administré dans la première dose de vaccination qui déclenche la réponse immunitaire ainsi que dans au moins l'une des doses de vaccination après la première.

17. Utilisation selon la revendication 15, dans laquelle le médicament est destiné à être administré dans seulement la première dose de vaccination qui déclenche la réponse immunitaire, les doses de vaccination après la première étant absentes.

18. Utilisation selon la revendication 17, dans laquelle le médicament est destiné à être administré dans seulement la première dose de vaccination qui déclenche la réponse immunitaire, au moins l'une des doses de vaccination subséquentes à la première dont le médicament est absent contenant un vecteur recombinant différent qui est également un système d'expression de la protéine LACK ou d'un fragment immunogène de celle-ci.

19. Utilisation selon la revendication 15, dans laquelle le médicament destiné à être administré dans au moins l'une des doses de vaccination subséquentes à la première, est absent de la première dose de vaccination.

20. Utilisation selon la revendication 19, dans laquelle le médicament est destiné à être administré dans au moins l'une des doses de vaccination subséquentes à la première, la première dose de vaccination, qui ne contient pas le médicament, contient un vecteur recombinant différent qui est également un système d'expression de la protéine LACK ou d'un fragment immunogène de celle-ci.

21. Utilisation selon la revendication 20, dans laquelle le médicament est destiné à être administré dans au moins l'une des doses de vaccination subséquentes à la première, la première dose de vaccination, qui ne contient pas le médicament, contient un ADN nu qui est également un système d'expression de la protéine LACK ou d'un fragment immunogène de celle-ci.

22. Utilisation selon la revendication 7, dans laquelle le mammifère susceptible d'être infecté par une espèce du genre *Leishmania* pour laquelle le médicament est destiné, est un chien.

23. Utilisation selon la revendication 22, dans laquelle la leishmaniose est provoquée par *Leishmania infantum.*

Figure 1

Figure 2

Anti-LACK          Anti-WR

Figure 3

Figure 4

**A**

**B**

Figure 5

**A**

Figure 6

Figure 7

Figure 8

A

DNA-LACK    DNA-LACK    DNA-LACK   DNA- CTRL

VV-LACK-TK⁻   VV-LACK-HA⁻   MVA-LACK-HA⁻   VV-LUC-HA⁻

B

DNA-LACK    DNA-LACK    DNA-LACK   DNA- CTRL

VV-LACK-TK⁻   VV-LACK-HA⁻   MVA-LACK-HA⁻   VV-LUC-HA⁻

Figure 9

Figure 10a

Figure 10b

Figure 11

**A**

Legend:
- pMOK / pMOK
- MIDGE-NLS-CONTROL / VV-LUC
- pMOCK-LACK / MVA-LACK

**B**

Figure 12

Figure 13

Figure 14

**A**

DNA_LACK / MVA_LACK 1 × 10$^7$

**D**

DNA CTRL / VV_LUC 5 × 10$^7$

**B**

DNA_LACK / MVA_LACK 5 × 10$^7$

**E**

N

**C**

DNA_LACK / VV_LACK 5 × 10$^7$

Figure 15

**Figure 16**

Figure 17

A

INTRACEL

B

EXTRACEL

Figure 18

A

B

**Figure 19**

**A**

**B**

**C**

Figure 20

IgG1 anti-SLA

C (-)
C (+)
DNA-LACK/rVV-LACK
DNA-LACK/MVA-LACK

time (days)

IgG2 anti-SLA

C (-)
C (+)
DNA-LACK/rVV-LACK
DNA-LACK/MVA-LACK

time (days)

Figure 21

EP 1 916 306 B1

51

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- ES 2171360

- ES 2172482

### Non-patent literature cited in the description

- **Ahmed, S. B. ; C. Bahloul ; C. Robbana ; S. Askri ; K. Dellagi.** A comparative evaluation of different DNA vaccine candidates against experimental murine leishmaniasis due to L. major. *Vaccine,* 2004, vol. 22, 1631-9
- **Altenburger, W. ; C. P. Suter ; J. Altenburger.** Partial deletion of the human host range gene in the attenuated vaccinia virus MVA. *Arch Virol,* 1989, vol. 105, 15-27
- **Berrahal, F. ; C. Mary ; M. Roze ; A. Berenger ; K. Escoffier ; D. Lamouroux ; S. Dunan.** Canine leishmaniasis: identification of asymptomatic carriers by polymerase chain reaction and immunoblotting. *Am J Trop Med Hyg,* 1996, vol. 55, 273-7
- **Blanchard, T. J. ; A. Alcami ; P. Andrea ; G. L. Smith.** Modified vaccinia virus Ankara undergoes limited replication in human cells and lacks several immunomodulatory proteins: implications for use as a human vaccine. *J Gen Virol,* 1998, vol. 79, 1159-67
- **Caver, T. E. ; T. D. Lockey ; R. V. Srinivas ; R. G. Webster ; J. L. Hurvitz.** A novel vaccine regimen utilizing DNA, vaccinia virus and protein immunizations for HIV-1 envelope presentation. *Vaccine,* 1999, vol. 17, 1567-72
- **Drexler, I. ; K. Heller ; B. Wahren ; V. Erfle ; G. Sutter.** Highly attenuated modified vaccinia virus Ankara replicates in baby hamster kidney cells, a potential host for virus propagation, but not in various human transformed and primary cells. *J Gen Virol,* 1998, vol. 79, 347-52
- **Gonzalez-Aseguinolaza, G. ; S. Taladriz ; A. Marquet ; V. Larraga.** Molecular cloning, cell localization and binding affinity to DNA replication proteins of the p36/LACK protective antigen from Leishmania infantum. *Eur J Biochem,* 1999, vol. 259, 909-16
- **Gonzalo, R. M. ; G. del Real ; J. R. Rodriguez ; D. Rodriguez ; R. Heljasvaara ; P. Lucas ; V. Larraga ; M. Esteban.** A heterologous prime-boost regime using DNA and recombinant vaccinia virus expressing the Leishmania infantum P36/LACK antigen protects BALB/c mice from cutaneous leishmaniasis. *Vaccine,* 2002, vol. 20, 1226-31

- **Gonzalo, R. M. ; J. R. Rodriguez ; D. Rodriguez ; G. Gonzalez-Aseguinolaza ; V. Larraga ; M. Esteban.** Protective immune response against cutaneous leishmaniasis by prime/booster immunization regimens with vaccinia virus recombinants expressing Leishmania infantum p36/LACK and IL-12 in combination with purified p36. *Microbes Infect,* 2001, vol. 3, 701-11
- **Gradoni, L.** An update on antileishmanial vaccine candidates and prospects for a canine Leishmania vaccine. *Vet Parasitol,* 2001, vol. 100, 87-103
- **Gurunathan, S. ; D. L. Sacks ; D. R. Brown ; S. L. Reiner ; H. Charest ; N. Glaichenhaus ; R. A. Seder.** Vaccination with DNA encoding the immunodominant LACK parasite antigen confers protective immunity to mice infected with Leishmania major. *J Exp Med,* 1997, vol. 186, 1137-47
- **Hanke, T. ; T. J. Blanchard ; J. Schneider ; C. M. Hannan ; M. Becker ; S. C. Gilbert ; A. V. Hill ; G. L. Smith ; A. McMichael.** Enhancement of MHC class I-restricted peptide-specific T cell induction by a DNA prime/MVA boost vaccination regime. *Vaccine,* 1998, vol. 16, 439-45
- **Hanke, T. ; A. McMichael.** Pre-clinical development of a multi-CTL epitope-based DNA prime MVA boost vaccine for AIDS. *Immunol Lett,* 1999, vol. 66, 177-81
- **Hanke, T. ; V. C. Neumann ; T. J. Blanchard ; P. Sweeney ; A. V. Hill ; G. L. Smith ; A. McMichael.** Effective induction of HIV-specific CTL by multi-epitope using gene gun in a combined vaccination regime. *Vaccine,* 1999, vol. 17, 589-96
- **Kent, S. J. ; A. Zhao ; S. J. Best ; J. D. Chandler ; D. B. Boyle ; I. A. Ramshaw.** Enhanced T-cell immunogenicity and protective efficacy of a human immunodeficiency virus type 1 vaccine regimen consisting of consecutive priming with DNA and boosting with recombinant fowlpox virus. *J Virol,* 1998, vol. 72, 10180-8

- **Kim, J. J. ; M. L. Bagarazzi ; N. Trivedi ; Y. Hu ; K. Kazahaya ; D. M. Wilson ; R. Ciccarelli ; M. A. Chattergoon ; K. Dang ; S. Mahalingam.** Engineering of in vivo immune responses to DNA immunization via codelivery of costimulatory molecule genes. *Nat Biotechnol,* 1997, vol. 15, 641-6

- **Lanotte, G. ; J. A. Rioux ; J. Perieres ; Y. Vollhardt.** Ecology of leishmaniasis in the south of France. 10. Developmental stages and clinical characterization of canine leishmaniasis in relation to epidemiology. (author's transl). *Ann Parasitol Hum Comp,* 1979, vol. 54, 277-95

- **Li, S. ; M. Rodrigues ; D. Rodriguez ; J. R. Rodriguez ; M. Esteban ; P. Palese ; R. S. Nussenzweig ; F. Zavala.** Priming with recombinant influenza virus followed by administration of recombinant vaccinia virus induces CD8+ T-cell-mediated protective immunity against malaria. *Proc Natl Acad Sci U S A,* 1993, vol. 90, 5214-8

- **Liew, F. Y. ; C. A. O'Donnell.** Immunology of leishmaniasis. *Adv Parasitol,* 1993, vol. 32, 161-259

- **Lopez-Fuertes, L. ; E. Perez-Jimenez ; A. J. Vila-Coro ; F. Sack ; S. Moreno ; S. A. Konig ; C. Junghans ; B. Wittig ; M. Timon ; M. Esteban.** DNA vaccination with linear minimalistic (MIDGE) vectors confers protection against Leishmania major infection in mice. *Vaccine,* 2002, vol. 21, 247-57

- **Lopez-Velez, R. ; J. A. Perez-Molina ; A. Guerrero ; F. Baquero ; J. Villarrubia ; L. Escribano ; C. Bellas ; F. Perez-Corral ; J. Alvar.** Clinicoepidemiologic characteristics, prognostic factors, and survival analysis of patients coinfected with human immunodeficiency virus and Leishmania in an area of Madrid, Spain. *Am J Trop Med Hyg,* 1998, vol. 58, 436-43

- **Mauricio, I. L. ; J.R Stothard ; M. A. Miles.** The strange case of Leishmania chagasi. *Parasitol Today,* 2000, vol. 16, 188-9

- **Mayr, A. ; H. Stickl ; H. K. Muller ; K. Danner ; H. Singer.** The smallpox vaccination strain MVA: marker, genetic structure, experience gained with the parenteral vaccination and behavior in organisms with a debilitated defence mechanism (author's transl). *Zentralbl Bakteriol [B],* 1978, vol. 167, 375-90

- **McMahon-Pratt, D. ; D. Rodriguez ; J. R. Rodriguez ; Y. Zhang ; K. Manson ; C. Bergman ; L. Rivas ; J. F. Rodriguez ; K. L. Lohman ; N. H. Ruddle et al.** Recombinant vaccinia viruses expressing GP46/M-2 protect against Leishmania infection. *Infect Immun,* 1993, vol. 61, 3351-9

- **Meyer, H. ; G. Sutter ; A. Mayr.** Mapping of deletions in the genome of the highly attenuated vaccinia virus MVA and their influence on virulence. *J Gen Virol,* 1991, vol. 72, 1031-8

- **Paoletti, E.** Applications of pox virus vectors to vaccination: an update. *Proc Natl Acad Sci U S A,* 1996, vol. 93, 11349-53

- **Ramirez, J. C. ; M. M. Gherardi ; M. Esteban.** Biology of attenuated modified vaccinia virus Ankara recombinant vector in mice: virus fate and activation of B- and T-cell immune responses in comparison with the Western Reserve strain and advantages as a vaccine. *J Virol,* 2000, vol. 74, 923-33

- **Robinson, H. L. ; D. C. Montefiori ; R. P. Johnson ; K. H. Manson ; M. L. Kalish ; J. D. Lifson ; T. A. Rizvi ; S. Lu ; S. L. Hu ; G. P. Mazzara.** Neutralizing antibody-independent containment of immunodeficiency virus challenges by DNA priming and recombinant pox virus booster immunizations. *Nat Med,* 1999, vol. 5, 526-34

- **Sacks, D. ; N. Noben-Trauth.** The immunology of susceptibility and resistance to Leishmania major in mice. *Nat Rev Immunol,* 2002, vol. 2, 845-58

- **Sedegah, M. ; T. R. Jones ; M. Kaur ; R. Hedstrom ; P. Hobart ; J. A. Tine ; S. L. Hoffman.** Boosting with recombinant vaccinia increases immunogenicity and protective efficacy of malaria DNA vaccine. *Proc Natl Acad Sci U S A,* 1998, vol. 95, 7648-53

- **Solano-Gallego, L. ; J. Llull ; G. Ramos ; C. Riera ; M. Arboix ; J. Alberola ; L. Ferrer.** The Ibizian hound presents a predominantly cellular immune response against natural Leishmania infection. *Vet Parasitol,* 2000, vol. 90, 37-45

- **Sutter, G. ; B. Moss.** Nonreplicating vaccinia vector efficiently expresses recombinant genes. *Proc Natl Acad Sci U S A,* 1992, vol. 89, 10847-51

- **Tapia, E. ; E. Perez-Jimenez ; L. Lopez-Fuertes ; R. Gonzalo ; M. M. Gherardi ; M. Esteban.** The combination of DNA vectors expressing IL- 12 + IL-18 elicits high protective immune response against cutaneous leishmaniasis after priming with DNA-p36/LACK and the cytokines, followed by a booster with a vaccinia virus recombinant expressing p36/LACK. *Microbes Infect,* 2003, vol. 5, 73-84

- **Xu, D. ; F. Y. Liew.** Genetic vaccination against leishmaniasis. *Vaccine,* 1994, vol. 12, 1534-6

- **Zavala, F. ; M. Rodrigues ; D. Rodriguez ; J. R. Rodriguez ; R. S. Nussenzweig ; M. Esteban.** A striking property of recombinant poxviruses: efficient inducers of in vivo expansion of primed CD8(+) T cells. *Virology,* 2001, vol. 280, 155-9

- **Melby, P.C. ; J. Yang ; W. Zhao ; L.E. Pérez ; J. Cheng.** Leishmania donovani p36(LACK) DNA vaccine is highly immunogenic but not protective against experimental visceral leishmaniasis. *Infect Immun,* 2001, vol. 69, 4719-4725

- **Moss, B.** Genetically engineered poxviruses for recombinant gene expression, vaccination, and safety. *Proc. Natl. Acad. Sci USA,* 1996, vol. 93, 11341-11348

- **Seki, M. ; M. Oie ; Y. Ichihashi ; H. Shida.** Hemadsoption and fusion inhibition activities of hemagglutinin analyzed by vaccinia virus mutants. *Virology,* 1990, vol. 175, 372-384

- **Chakrabarti, S. ; J.R. Sisler ; B. Moss.** Compact, synthetic, vaccina virus early/late promoter for protein expression. *BioTechniques,* 1997, vol. 23, 1094-1097

- **Belli, S. ; A. Formenton ; T. Noll ; A. Ivens ; R. Jacquet ; C. Desponds ; D. Hofer ; N. Fasel.** Leishmania major. histone H1 gene expression from the sw3 locus. *Exp. Parasitol,* 1999, vol. 91, 151-160

- **Campos-Neto, A. ; R. Porozzi ; K. Greeson ; R.M. Coler ; J.R. Webb ; Y.A. Seiky ; S.G. Reed ; G. Grimaldi, Jr.** Protection against cutaneous leishmaniasis induced by recombinant antigens in murine and nonhuman primate models of the human disease. *Infect. Immun.,* 2001, vol. 69, 4103-4108

- **Masina, S., M.G.M. ; S.O. Demotz ; N.J. Fasel.** Protection against cutaneous leishmaniasis in outbred brevet monkeys using a recombinat histone H1 antigen. *J. Infect. Dis.,* 2003, vol. 188, 1250-1257

- **Ahmed, S. ; M. Colmenares ; L. Soong ; K. Goldsmith-Pestana ; L. Munstermann ; R. Molina ; D. McMahon-Pratt.** Intradermal infection model for patogénesis and vaccine studies of murine visceral leishmaniasis. *Infect Immun,* 2003, vol. 71, 401-10

- **Bhakuni, V. ; U.K Singha ; G.P. Dutta ; H.B. Levy ; R.K. Maheshawari.** Killing of Leishmania donovani amastigotes by poly ICLC in hamsters. *J. Interferon Cytokine Res.,* 1996, vol. 16, 321-325

- **Green, S.J., M.S. ; Meltzer, J.B. ; Hibbs, Jr. ; C.A, Nacy.** Activated macrophages destroy intracellular Leishmania major amastigotes by and L-arginine-dependent killing mechanism. *J. Immunol,* 1990, vol. 144, 278-283

- **Liew, F. ; Y.S. Millot ; C. Parkinson ; R.M. Palmer ; S. Moncada.** Macropage killing of Leishmania parasite in vivo is mediated by nitric oxide from L-arginine. *J. Immunol.,* 1990, vol. 144, 4791-4797

- **Martínez-Moreno, A ; Martinez-Cruz MS ; Blanco A ; Hernández-Rodriguez S.** Inmunological histological study of T-and- B-lymphocyte activity in canine visceral leishmaniasis. *Vet. Parasitol,* 1993, vol. 51, 49-59

- **Riera, C ; Valladares JE ; Gállego M et al.** Serological and parasitological follow-up in dogs experimentally infected with Leishmania infantum and treated with menglubine antimoniate. *Vet Parasitol,* 1999, vol. 84, 33-47

- WHO Manual on visceral leishmaniasis control. *WHO/LEISH/96.40. Ginebra (Suiza),* 51-70

- **Boelaert M ; El Safi S ; Mousa H et al.** Multi-centre evaluation of repeatability and reproducibility of the direct agglutination test for visceral leishmaniasis. *Trop Med Int Health,* 1999, vol. 4 (1), 31-7

- **Boelaert M ; El Safi S ; Jacquet D ; Van der Stuyf P ; Le Ray D ; De Muynck D.** Operational validation of the direct agglutination test for diagnosis of visceral leishmaniasis. *Am J Trop Med Hyg,* 1999, vol. 4 (1), 129-34

- **Boelaert M ; El Safi S ; Goethebeur E ; Gomes-Pereira S ; Le Ray D ; van der Stuyf P.** Latent class analysis permits unbiased estimates of the validity of DAT for the diagnosis of visceral leishmaniasis. *Trop Med Int Health,* 1999, vol. 4 (5), 395-401

- **Baumann RJ ; Hanson WL ; McCaan PP ; Sjodersma A ; Bionti AJ.** Suppression of both antimony-susceptible and antimony-resistant Leishmania donovani by a bis(benzyl)polyamine analogue. *Antimicrob Agents Chemother,* 1990, vol. 34, 722-7

- **Tesouro MA et al.** *Información Veterinari,* 1992, vol. 127, 34-40

- **Ashford DA et al.** *Am. J. Trop. Med. Hyg.,* 1995, vol. 53, 251-255